# EUROPÄISCHE PATENTSCHRIFT

(11) **EP 1 292 230 B1**
(45) Veröffentlichungstag und Bekanntmachung des Hinweises auf die Patenterteilung: **26.09.2007**
(21) Anmeldenummer: 01936388.6
(22) Anmeldetag: 22.05.2001
(51) Int. Cl.: A61B 10/00

(54) **CHIRURGISCHE HOHLSONDE**
SURGICAL, GROOVED DIRECTOR
SONDE CHIRURGICALE CREUSE

(30) Priorität: 24.05.2000 DE 10026508
(43) Veröffentlichungstag der Anmeldung: 19.03.2003
(73) Patentinhaber: Desinger, Kai, Dr., 12157 Berlin (DE)
(72) Erfinder: Desinger, Kai, Dr., 12157 Berlin (DE)
(74) Vertreter: Eisenführ, Speiser & Partner
(86) Internationale Anmeldenummer: PCT/EP2001/005880
(87) Internationale Veröffentlichungsnummer: WO 2001/089388

(56) Entgegenhaltungen:
- EP-A- 0 761 170
- EP-A- 0 919 192
- WO-A-96/14018
- WO-A-98/08441
- US-A- 5 749 869
- US-A- 6 050 955

## Beschreibung

Die Erfindung betrifft eine chirurgische Hohlsonde zur minimal invasiven Entnahme von Gewebe mit einem länglichen Hohlkörper, welcher zwei Segmente umfasst, von denen zumindest eines einen Hohlraum zur Aufnahme von Gewebe einschließt und welche voneinander trennbar und relativ zueinander derart bewegbar angeordnet sind, dass eine Öffnung zwischen den Segmenten wahlweise freizugeben oder zu verschließen ist.

Im Rahmen der minimal invasiven Medizin wird versucht, ein für den Patienten minimal traumatisierendes Operieren zu verwirklichen. Dabei wird versucht, entweder über die natürlichen Körperöffnungen des Patienten, wie bei der interventionellen Endoskopie über Instrumente (Endoskope) mit Sichtkontrolle, oder aber durch kleine Inzisionen direkt an die Eingriffsstelle in die Tiefe des Gewebes zu gelangen, um dort Gewebe für die Diagnostik, beispielsweise zur histologischen Gewebebestimmung oder zur Therapie, z.B. zur Tumorentfernung, zu entnehmen.

Die Entwicklung entsprechender Instrumente für die minimal invasive Chirurgie schreitet in den letzten Jahren rasch voran, da diese Operationstechnik aus kosmetischen Gründen, zur Minimierung postoperativer Komplikationen und zur Beschleunigung des Heilungsprozesses erhebliche Vorteile beinhaltet.

Unter der Bezeichnung "Feinnadelaspiration" (FNA) oder "True-Cut-Nadelbiopsie" sind Vorrichtungen bekannt, die eine feine Nadel aufweisen, welche in ein verdächtiges Gewebeareal eingebracht wird, und mittels der sich eine oder mehrere Gewebeproben rein mechanisch über einen Stanzmechanismus entnehmen lassen, um dieses Gewebe histologisch zu untersuchen.

Danach können gegebenenfalls bei einem bösartigen Befund entsprechende chirurgische Maßnahmen eingeleitet werden. Die mit diesen bekannten Nadelsonden entnehmbaren Gewebevolumina sind jedoch nur sehr klein und eignen sich daher ausschließlich für diagnostische Zwecke. Aufgrund des geringen Gewebevolumens, welches sich bei einem Einstich entnehmen lässt, sind damit oftmals mehrere Einstiche in das verdächtige Gewebeareal erforderlich, was unter Umständen zu einer Zellverschleppung von bösartigen Tumorzellen führen kann. Des Weiteren sind aufgrund des kleinen Gewebevolumens die Genauigkeiten des histologischen Gewebebefundes nicht optimal.

Aus der US 5,775,333 oder der US 5,782,795 sind chirurgische Instrumente bekannt, die eine Entnahme größerer Gewebevolumina durch multiple Gewebeentnahme aus dem gleichen Zielareal ermöglichen. Bei diesen bekannten Instrumenten kommt eine vakuumunterstützte mechanische Stanz-Schneidvorrichtung zum Einsatz, welche die Anzahl der erforderlichen Einstiche reduziert und außerdem - wegen des größeren Gewebevolumens - auch die Genauigkeit des diagnostischen Befundes verbessert. Mit diesen bekannten Instrumenten lassen sich auch kleinere Gewebeveränderungen oftmals schon intoto entfernen, was unter Umständen eine Nachoperation entbehrlich macht.

Bei dem Instrument gemäß der US 5,775,333 wird eine nadelförmige Hohlsonde in das Zielgebiet, beispielsweise einen Brusttumor, eingebracht. Am Ende dieser Sonde befindet sich seitlich eine längliche Öffnung, in welche das zu entnehmende Gewebe eingesaugt wird. Innerhalb der Vorrichtung befindet sich ein rotierendes Hohlmesser, welches über einen mechanischen Vorschub das in die Öffnung eingesogene Gewebe innerhalb der Vorrichtung abschneidet und durch ein Vakuum durch die Hohlsonde hindurch absaugt. Durch sukzessive Drehung des Schaftes um 360° wird so ein bestimmtes in die Hohlsonde zuvor eingesaugtes und abgeschnittenes Gewebevolumen um das distale Ende der Sonde herum entnommen, was jedoch für eine komplette Entfernung des medizinisch verdächtigen Gewebeareals oft nicht gänzlich ausreicht.

Aus der US-PS 5,817,034 ist ein chirurgisches Instrument bekannt, bei dem ein Rohrzylindermesser mit einem Durchmesser bis zu 25 mm zum Zielgebiet vorangetrieben wird. Über eine am Ende befindliche, mit HF-Spannung beaufschlagte Schlinge wird dann das Kopfstück des so innerhalb der Vorrichtung ausgestanzten Zylinders abgetrennt und anschließend der gesamte abgetrennte Gewebezylinder über die Vorrichtung entnommen. Bei diesem bekannten System kann zwar das verdächtige Gewebeareal in toto entnommen werden, es wird jedoch auch sehr viel gesundes Gewebe entfernt, bis die Sondenspitze das verdächtige Gewebeareal erreicht hat, und es bleibt dann eine relativ große Narbe durch den großen Vorrichtungsdurchmesser zurück.

Aus der US 5,810,806 ist eine chirurgische Sonde bekannt, bei der das Gewebe nicht mittels eines mechanischen Ringmessers, sondern mit Hilfe einer am distalen Ende des Hohlkörpers befestigten, axial unverschiebbaren Schlinge eingeschnitten wird, die mit einer HF-Spannung beaufschlagt wird und dadurch als Schneidmesser wirkt. Bei einem Schneidvorgang schiebt sich der am Umfang von der HF-Schlinge abgetrennte Gewebezylinder frontal in den vorne offenen Hohlkörper, und wird dann am Ende der Prozedur durch eine scheibenwischerartige Drehbewegung der mit HF-Spannung beaufschlagten Schlinge abgetrennt. Auch bei dieser bekannten Vorrichtung wird das Gewebe erst dann vollständig abgetrennt, wenn es sich in dem Hohlkörper befindet, wodurch auch viel gesundes Gewebe mit entfernt wird, bis die Sondenspitze bis zum verdächtigen Gewebeareal vorgeschoben ist. Nachteilig ist außerdem, dass nur Gewebezylinder vor der Frontöffnung des Hohlkörpers entfernt werden könne.

Aus der DE 197 06 751 oder der DE 195 28 440 ist ein elektrochirurgisches Gerät der eingangs genannten Art bekannt, bei dem ein schlaufenförmiges Schneidelement zum elektrochirurgischen Ausschneiden von Gewebe parallel zur Längsachse eines länglichen Hohlkörpers ausgerichtet ist und unter Beibehaltung dieser Ausrichtung schlaufenförmig aus dem Hohlkörper ausfahrbar ist, damit dann das angrenzende Gewebe durch Drehung der Hohlsonde torusförmig ausgeschnitten werden kann. Das ausgeschnittene Gewebe lässt sich dann durch dieselbe Öffnung, durch welche das Schneidelement austritt, in den Hohlkanal des Hohlkörpers hineinschleusen und dann längs des Hohlkanals zum proximalen Ende des Geräts transportieren. Dieses bekannte Gerät hat den Vorteil, dass die Einstichstelle geringe Abmessungen aufweist, und dass dann an dem Behandlungsort ein Gewebe entnommen werden kann, welches die Hohlsonde außen angrenzend umgibt. Nachteilig ist dabei jedoch, dass das ausschneidbare Gewebevolumen relativ klein ist und daher zu einer gründlichen Untersuchung auch größerer Gewebebereiche nicht ausreicht.

Aus der nachveröffentlichten WO 99/44506 ist ein schlaufenförmiges Schneidelement bekannt, welches in einer Ebene aus dem Hohlkörper ausfahrbar ist, die quer zur Längsachse des Hohlkörpers verläuft, wobei nach dem Ausfahren des Schneidelements das Schneidelement längs des Hohlkörpers verschiebbar ist.

Aus der US 5,810,659 ist eine chirurgische Sonde zur Gewebeentnahme bekannt, bei der am distalen Ende eines Schaftes eine scharf angeschliffene Schneidhülse sitzt, welche mit einer HF-Spannung beaufschlagt werden kann. Distal hinter der Schneidhülse, an einem zentral durch den Katheter laufenden Führungsdraht befindet sich ein Keramikkörper als Gegenlager zur Schneidhülse, an dessen Ende eine abgerundete Metallelektrode befestigt und über den Führungsdraht mit einem HF-Generator verbunden ist. Über ein Bedienteil kann das zylinderförmige Gegenlager an die scharf angeschliffene Metallhülse über den Führungsdraht herangezogen werden, wobei das dazwischenliegende Gewebe durch den scharfen Anschliff der Metallhülse rein mechanisch abgetrennt wird und sich so innerhalb der proximalen Schneidhülse sammelt. Sowohl die proximale Hülse als auch die am Gegenlager befestigte Metallelektrode sind mit einem HF-Generator verbunden, und ermöglichen so ein bipolares Koagulieren von Gewebe um ggf. auch Blutungen zu stillen. Hierbei gibt es jedoch keine technische Lösung zur einfachen Entnahme des einmal gesammelten Biopsats. Das Gewebe muss mit einer Mikro-Pinzette oder Nadel aus der Hülse umständlich entnommen werden.

Aus der Anmeldung PCT/US99/21416 ist ein starres chirurgisches Instrument bekannt, was ebenfalls aus einer zueinander verschiebbaren Hülsenkonfiguration besteht, um Gewebeproben zu entnehmen und das Gewebe zu koagulieren. Die Neuerung zum Stand der Technik liegt zum einen in dem aufwendigen, teilautomatisierten Bedienteil, sowie in der Möglichkeit über das Anlegen eines Schneidstromes an eine der beiden verfahrbaren Hülsen, um das Gewebe elektrochirurgisch abzuschneiden. In einer weiteren Ausführungsform übernimmt das Schneiden nicht die Hülse selbst, sondern eine in der Ummantelung am proximalen Hülsenumfang eingelassene feststehende Schneidelektrode.

Weiterhin ist aus EP 0 919192 A2 ein chirurgisches Instrument bekannt, welches an seinem distalen Ende zwei axial zueinander verschiebbare Hülsen aufweist, welche auseinander gefahren werden können und dabei einen Hohlraum zur Gewebeaufnahme freigeben. Beim Zusammenschieben der Hülsen wird durch ein an dem distalen Ende der proximalen Hülle angebrachtes Schneidelement das in den Hohlraum eingedrungenes Gewebe, welches zusätzlich durch ein angelegtes Vakuum in den Hohlraum eingesogen werden kann, abgeschnitten. Das Gewebe, das sich nach dem Schneidvorgang in dem Hohlraum befindet, kann nun durch herausziehen der gesamten Sonde entfernt werden.

Aufgabe der Erfindung ist es, eine chirurgische Sonde der eingangs genannten Art anzugeben, welche in der Vermeidung der Nachteile des Standes der Technik zuverlässig und einfach handhabbar ist.

Gelöst wird diese Aufgabe durch eine chirurgische Hohlsonde gemäß Anspruch 1, die ein elektrisch leitendes, relativ zu zumindest einem der Segmente bewegbares ring- oder schlaufenförmiges Schneidelement aufweist, welches zum elektrochirurgischen Ausschneiden von in die Öffnung zwischen den Halbkörpersegmenten eingedrungenem Gewebe ausgebildet ist, sowie zusätzlich einen Auswerfer zum Auswerfen von Gewebe aus der Hohlsonde.

Eine derartige Hohlsonde erlaubt es insbesondere, das Schneidelement in eine besonders bevorzugte freistehende Position zu bewegen. Wenn dann an das Schneidelement wie vorgesehen eine Hochfrequenzspannung angelegt wird, kommt es aufgrund der Feldkonzentration um das Schneidelement zur Erwärmung des unmittelbar an das Schneidelement angrenzenden Gewebes. Bei freistehendem Schneidelement kann diese Erwärmung soweit gehen, dass sich um das Schneidelement ein Dampfpolster bildet, so dass das Schneidelement praktisch berührungslos durch das Gewebe dringen kann. Es hat sich gezeigt, dass ein derartiger Effekt, bei dem das Schneidelement vollständig von einem Dampfpolster umgeben ist, bei einer in das Frontende einer Hülse eingelassenen Elektrode, wie sie aus der PCT/US99/21416 bekannt ist, nicht oder nur bei größeren Feldstärken auftritt.

Die als Drahtschlaufe oder Drahtschlinge ausgebildete Schneidelektrode erzeugt durch die angelegte Hochfrequenzspannung (> 200 V) viele kleine Funkenentladungen entlang der Elektrode. Damit wird ein Dampfpolster zwischen Gewebe und Elektrode erzeugt, was ein quasi berührungsfreies, elektrisches Schneiden erlaubt. Durch die Verwendung eines eher sinusförmigen hochfrequenten Wechselstromes können Schnitte im Gewebe mit minimaler thermischer Randkoagulation erzeugt werden. Durch Änderung des sogenannten Crest-Faktors, also dem Verhältnis von Spitzenspannung zu Effektivspannung sowie zusätzlicher HF-Modulation, lassen sich verschiedene Koagulationsgrade an der Schnittfläche einstellen. Die Rückleitung des Stromes erfolgt in einer erfindungsgemäßen Ausführung über eine oder mehrere Funktionsflächen an dem Hohlkörper. Mit dieser bipolaren Elektrodenkonfiguration lassen sich patientensichere und schmerzarme Eingriffe mit geringer Generatorleistung durchführen. Aber auch als monopolare Ausführung, mit an die Patientenextremität anlegbarer Rückleitelektrode, ist diese Sonde zu betreiben.

Weiterhin erlaubt es die erfindungsgemäße Hohlsonde, entnommenes Gewebe auf einfache Weise auszuwerfen. Dem dient ein in die Sonde integrierter Auswerfer, so dass zum Entnehmen von Gewebe aus der Hohlsonde kein gesondertes Werkzeug erforderlich ist.

Vorzugsweise sind die beiden Segmente der Hohlsonde entlang einer Umfangslinie um den Hohlraum voneinander trennbar. Die beiden Segmente sind dann vorzugsweise axial zueinander verschiebbar. In einer bevorzugten Ausführungsform verläuft die Trennlinie zwischen den beiden Segmenten nahe dem distalen Ende der Hohlsonde. Bei einer Hohlsonde wird vorzugsweise ein Segment von der Spitze der Hohlsonde gebildet, während das zweite Segment eine bezüglich der Spitze axial verschiebbare Hülse mit rundem, ovalem oder vieleckigem Querschnitt ist. Nach dem Einstechen der Hohlsonde in Gewebe kann eine verschiebbare Hülse zurückgezogen werden, so dass sich je nach Querschnittsform der Hülse eine zylinder- oder prismenförmige Öffnung zwischen der Spitze der Hohlsonde und der Hülse ergibt.

Vorzugsweise wird diese Verschiebbarkeit durch eine Schubstange erzielt, welche mit der Spitze fest verbunden und in der Hülse längsgeführt ist. Die Schubstange kann entweder mittig in einem von der Hülse eingeschlossenen Hohlraum verlaufen, oder nahe der Hülsenwandung.

Die erstgenannte Variante hat den Vorteil einer symmetrischen Kraftverteilung, während die zweitgenannte Variante den Vorteil hat, dass der von der Hülse definierte Hohlraum die Schubstange nicht umschließt, sondern sich die Schubstange ganz auf einer Seite des Hohlraumes befindet. Dies bietet Vorteile beim Auswerfen von mit der Hohlsonde entnommenem Körpergewebe.

Besonders bevorzugt läuft das distale Ende der Hohlsonde spitz zu und trägt eine erste Elektrode, die beim Ausschneiden von Gewebe als Gegenelektrode für das Schneidelement dienen kann. Bei dem Schneidbetrieb wird dabei ein Anschluss der HF-Spannungsquelle an das Schneidelement, der andere Anschluss an die erste Elektrode gelegt. Beim Einstechen der Sonde liegt das Schneidelement passiv ohne Spannungsbeaufschlagung im Innern des Hohlkörpers.

Das Schneidelement wird vorzugsweise von einer runden, ovalen oder vieleckig geformten Drahtschlaufe gebildet, welche eine Ebene definiert und senkrecht zu dieser Ebene relativ zu zumindest einem der Segmente axial verschiebbar mittels vorzugsweise einer Schubstange geführt ist. Eine solche Drahtschlaufe hat die Eigenschaft, elektrisch leitend zu sein und kann so als eine Elektrode für das Anlegen einer elektrischen Hochfrequenzspannung dienen. Die Gegenelektrode kann beispielsweise das von der Hohlsondenspitze gebildete eine Segment oder das von der verschiebbaren Hülse gebildete andere Segment der Hohlsonde darstellen. Wenn der Draht für das Schneidelement einen Durchmesser zwischen 0,05 und 1 mm, vorzugsweise ca. 0,15 mm besitzt, kann die Gegenelektrode leicht eine wesentlich größere Oberfläche haben, als das Schneidelement, so daß sich ein elektrisches Feld zwischen Schneidelement und Gegenelektrode um das Schneidelement konzentriert. Diese erwünschte Feldkonzentration führt dazu, dass die zuvor beschriebene Bildung einer Dampfblase nur um das Schneidelement herum auftritt.

In einer bevorzugten Ausführungsform besteht die Drahtschlaufe oder Drahtschlinge aus einem ringförmig gebogenem Schneiddraht, welche über einen oder mehrere speichenförmig angeordnete Schneiddrähte mit einem zentralen, rohrförmigen Stab oder Hülse verbunden sind. Durch Vor- und Zurückfahren der zentralen Hülse kann die Ringelektrode durch das Gewebe geschoben sowie um die zentrale Achse gedreht werden, um Gewebe ab- bzw. einzuschneiden.

Insbesondere für den Fall, dass die die beiden Segmente verbindende Schubstange zentral im Hohlraum verläuft, ist es vorteilhaft, wenn zwischen der Drahtschlaufe und der Schubstange ein weiterer als Schneidelektrode dienender Draht vorgesehen ist, um beispielsweise einen torusartig die Schubstange umgreifenden Gewebeabschnitt auch an der Seite auftrennen zu können.

In einer solchen Ausführungsvariante ist die Schneidelektrode an ihrem Umfang mit einem rechtwinklig dazu angeordneten stabförmigen Körper als Schubstange verbunden, während die die beiden Segmente verbindende Schubstange zentral im wesentlichen durch den Mittelpunkt der Drahtschlaufe verläuft. Zum Einschneiden der zu entnehmenden, torusförmigen Gewebeproben dienen zwischen der als Drahtschlaufe ausgebildeten Ringelektrode und der zentralen Schubstange angebrachten Speichen oder Schlaufen, welche das spätere Entnehmen der Probe ermöglichen. Dabei liegt die Schubstange für die Drahtschlaufe verschiebbar gelagert innerhalb des Hohlkörpers und ist z. B. als Profilkörper ausgebildet oder durch andere bekannte Maßnahmen verdrehsicher gelagert. In dieser Ausführungsform ist die Schneidelektrode nicht verdrehbar.

Das Schneidelement wird über eine Anschlussleitung, die beispielsweise an der Innenwand des Hohlkörpers entlang geführt ist, an eine HF-Spannungsquelle angeschlossen, die sich am proximalen Ende der Hohlsonde befindet. Der andere Anschluss der HF-Spannungsquelle liegt an einer Gegenelektrode, die entweder von außen auf die Haut des Patienten aufgesetzt wird oder - um unkontrollierten Stromfluss durch das Gewebe zu verhindern - sich an dem Hohlkörper befindet bzw. Teil des Hohlkörpers ist. Wird der Hohlkörper aus Metall hergestellt, so kann auch der gesamte Hohlkörper der Hohlsonde als Gegenelektrode dienen, wodurch sich vom Schneidelement zu der Gegenelektrode ein kontrollierter, lokal eng begrenzter Stromfluss ergibt. Diese sind dann jedoch beide gut voneinander elektrisch zu isolieren, um einen Kurzschluss zu vermeiden.

Weiterhin sind die beiden Segmente vorzugsweise als Elektroden ausgebildet, an die Hochfrequenzspannung für das thermische Inaktivieren von Gewebe angelegt werden kann. Dazu kann sich zwischen den beiden Segmenten ein Isolatorelement befinden.

Besonders bevorzugt wird während des Einstechvorganges die vorzugsweise von der Metallspitze gebildete erste Elektrode mit HF-Spannung beaufschlagt, und eine Gegenelektrode wird in der Nähe des zu untersuchenden Gewebeareals von außen auf den Patienten aufgesetzt. Das an die Sonde angrenzende Gewebe wird dann mittels eines lokal begrenzten, hochfrequenten Wechselstroms über einen individuell vorgebbaren Zeitraum thermisch inaktiviert. Wird außerdem in vorgegebenem axialen Abstand eine zweite Elektrode auf den Hohlkörper aufgebracht, so lässt sich das hochfrequente Wechselfeld zwischen den beiden - bevorzugt zylinderförmigen Elektroden - erzeugen, wodurch das Wechselfeld - beim Einstechen der Sonde - lokal auf unmittelbar angrenzende Gewebezonen begrenzt ist. Die von außen aufgesetzte Gegenelektrode kann dann entfallen. Die HF-Spannung wird dabei so festgelegt, dass Temperaturen zwischen 55° bis 100°C, bevorzugt zwischen 60° und 80°C, über einen längeren Zeitraum von beispielsweise 5 bis 20 Minuten im Gewebe gehalten werden, um so die Stoffwechselvorgänge innerhalb der Tumorzellen irreversibel zu beenden. Durch diese thermische Inaktivierung wird gewährleistet, dass sich die Tumorzellen bei der anschließenden Gewebeentnahme nicht mehr verschleppen und metastasieren können.

Besonders bevorzugt ist eine elektrochirurgische Vorrichtung, die eine Hohlsonde der beschriebenen Art mit zwei an oder in unmittelbarer Nähe zu der Hohlsonde angeordneten Elektroden für das Einleiten hochfrequenten Stromes in das die Hohlsonde umgebende Gewebe umfasst, welche mit einer Vorrichtung zur Messung der Impedanz zwischen den beiden Elektroden verbunden ist. Eine solche Vorrichtung erlaubt es, während des Einleitens des hochfrequenten Stromes die Impedanz zwischen den beiden Elektroden zu messen. Dies kann durch die Erfassung der Stromstärke, Spannung oder des Fadenwinkels erfolgen. Außerdem lässt sich die effektiv ins Gewebe eingebrachte Leistung berechnen.

Durch die Impedanzmessung während des Einleitens des hochfrequenten Stromes lässt sich insbesondere der spezifische Gewebewiderstand desjenigen Gewebes bestimmen, welches die Hohlsonde umgibt. Anders als bei einer monopolaren Technik mit einer außen an den Körper angesetzten Gegen- oder Neutralelektrode, bei der der gesamte periphere Gewebewiderstand gemessen wird, wird bei der hier bevorzugten bipolaren Technik der reale, örtliche spezifische Gewebewiderstand zwischen den Elektroden gemessen. Die so gewonnenen Daten werden vorzugsweise einem Leistungs- oder Impedanzregler für einen Generator zugeführt, der dem Zeugen die in das Gewebe einzubringenden elektrischen Leistung dient. Durch die Impedanz oder gewebewiderstandsabhängige Regelung wird die Generatorleistung immer an den jeweils aktuellen Gewebestatus angepasst. Außerdem können die jeweils gewonnenen Daten auch als Information über den jeweils aktuellen Gewebestatus beispielsweise akustisch oder optisch angezeigt werden. Dies eröffnet einem Chirurg beispielsweise die Möglichkeit, die Behandlung mit der hier vorgestellten Hohlsonde individuell und aktuell dem jeweils rückgemeldeten Status des Gewebes anzupassen.

Der Auswerfer ist vorzugsweise ein nach Art eines Stempels im Hohlraum längsverschieblich angeordneter kolbenartiger Körper, der sich in seiner Ruheposition vorzugsweise an einem Ende des Hohlraumes befindet und aus dieser Ruheposition heraus in Richtung des Hohlraums verschiebbar ist, um in dem Hohlraum befindliches Gewebe bei durch Verschieben der Segmente geöffnetem Hohlraum auswerfen zu können. Falls solches Gewebe nach Art eines Torus beispielsweise eine Schubstange für eines der Segmente umgreifen sollte, können auf der dem Hohlraum zugeordneten Seite des Auswerfers ein oder mehrere Trennmesser vorgesehen sein, die das Gewebe beim Auswerfen derart schneiden, dass der Torus um die Schubstange nicht mehr geschlossen ist.

Gemäß einer bevorzugten Ausführungsform der Erfindung ist der Auswerfer ein innerhalb des Hohlraums im proximalen Teil des Hohlkörpers verschiebbar angeordnetes Kolbenelement, welches bezüglich der Metallhülse derart dichtend gelagert ist, dass nach dem Abtrennen des Gewebekörpers über das axial verfahrbare Schneidelement durch Zurückziehen des Kolbenelementes ein Vakuum erzeugt wird, welches das Einbringen des ausgeschnittenen Gewebezylinders oder -torus in die proximale Hülse des Hohlkörpers unterstützt.

In einer weiteren Ausführungsform kann das Vakuum alternativ oder zusätzlich durch eine separat angeschlossene Vakuumpumpe erzeugt und an den Hohlkörper zum Einziehen des Gewebes angelegt werden.

Vorteilhafte Weiterbildungen der Erfindung sind durch die Merkmale der Unteransprüche gekennzeichnet.

Im Folgenden werden Ausführungsbeispiele der Erfindung anhand der Zeichnungen näher erläutert. Es zeigen:
- Figuren 1 a bis 1l: Eine erste Ausführungsform einer Hohlsonde in verschiedenen Betriebszuständen
- Figuren 2 bis 9: alternative Ausführungsformen einer Hohlsonde in verschiedenen Betriebszuständen
- Figuren 10a bis 10h: zeigen verschiedene Ausführungsvarianten einer zentralen Schubstange mit daran befestigter Schneidschlaufe
- Figuren 11a bis 11h: zeigen alternative Ausführungsvarianten einer dezentralen Schubstange mit Schneidschlaufe
- Figur 12: zeigt eine alternative Hohlsonde in schematischer perspektivischer Darstellung
- Figuren 13 und 14: sind schematische Schnittansichten der Hohlsonde aus Figur 12 in verschiedenen Betriebszuständen
- Figur 15: zeigt eine Hohlsonde ähnlich der in Figur 3 in schematischer perspektivischer Darstellung
- Figur 16: zeigt eine chirurgische Vorrichtung mit Hohlsonde und HF-Generator

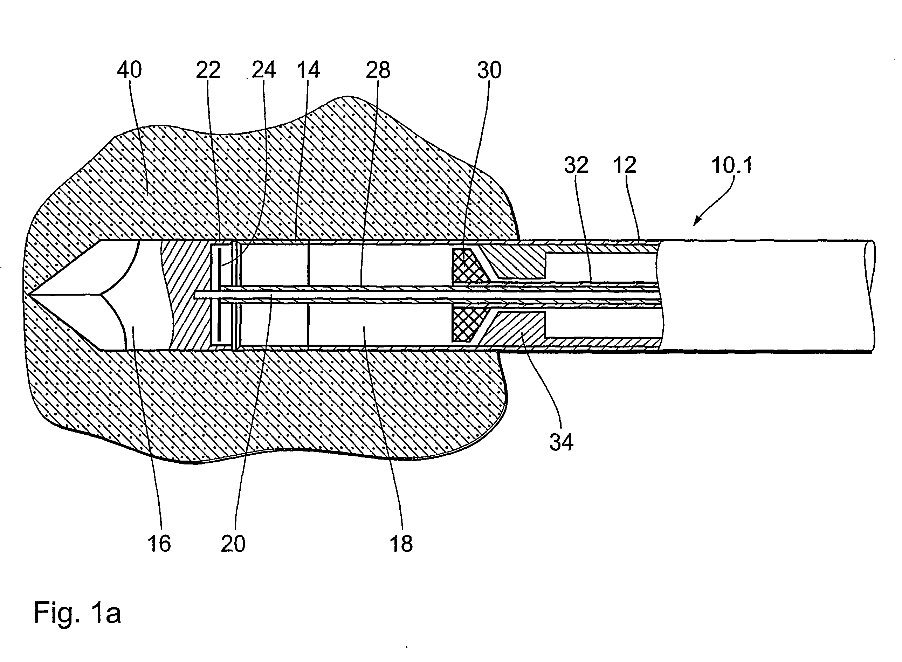

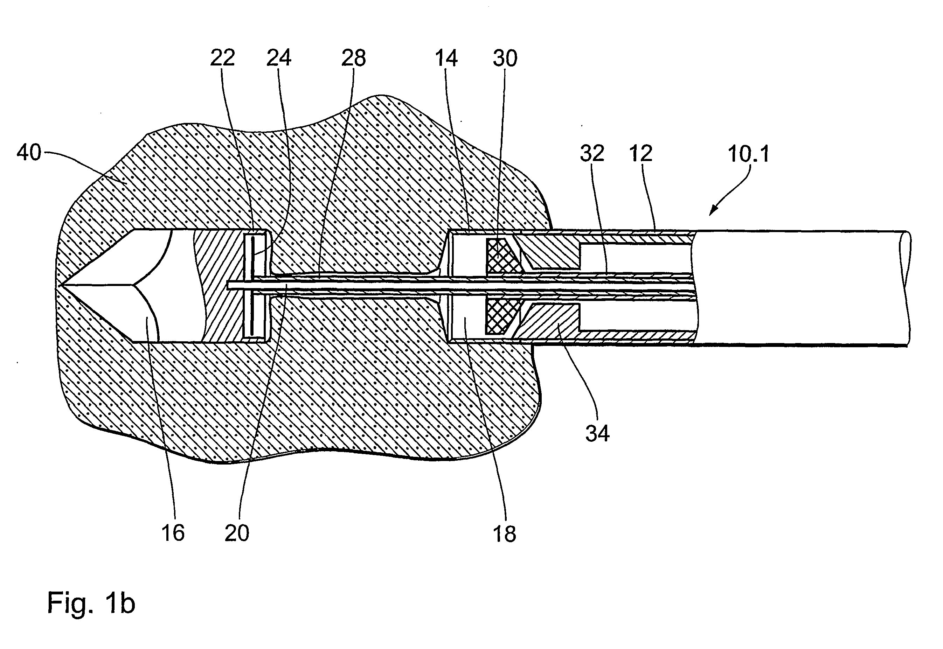

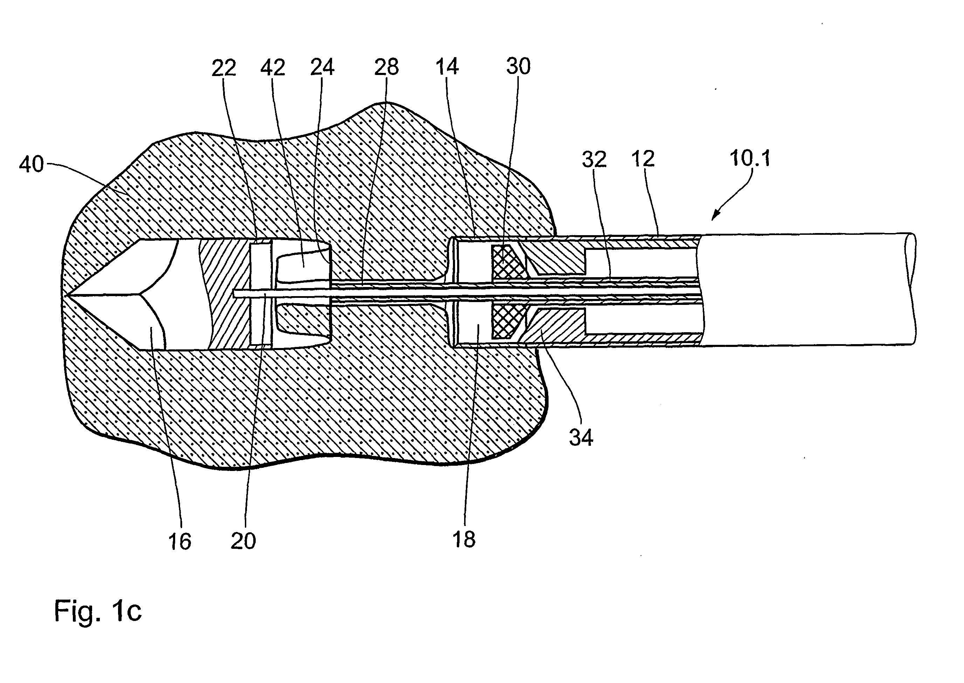

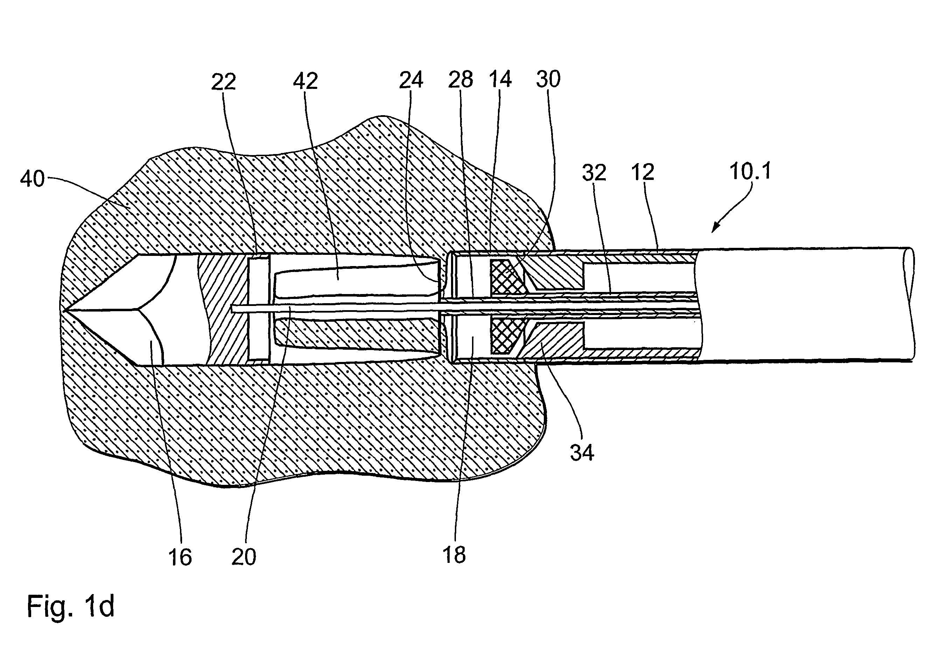

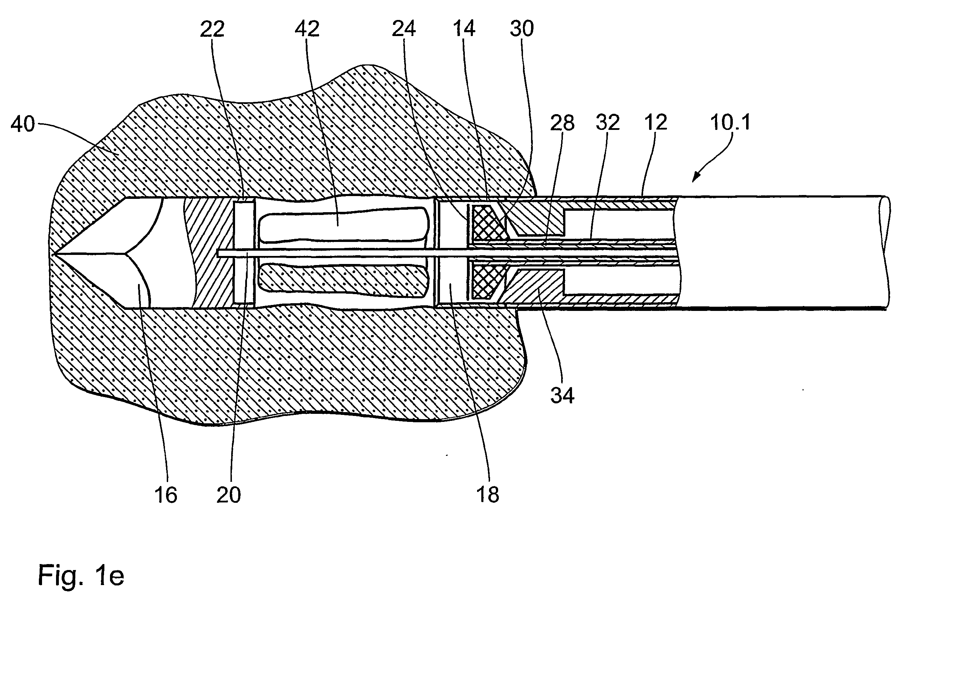

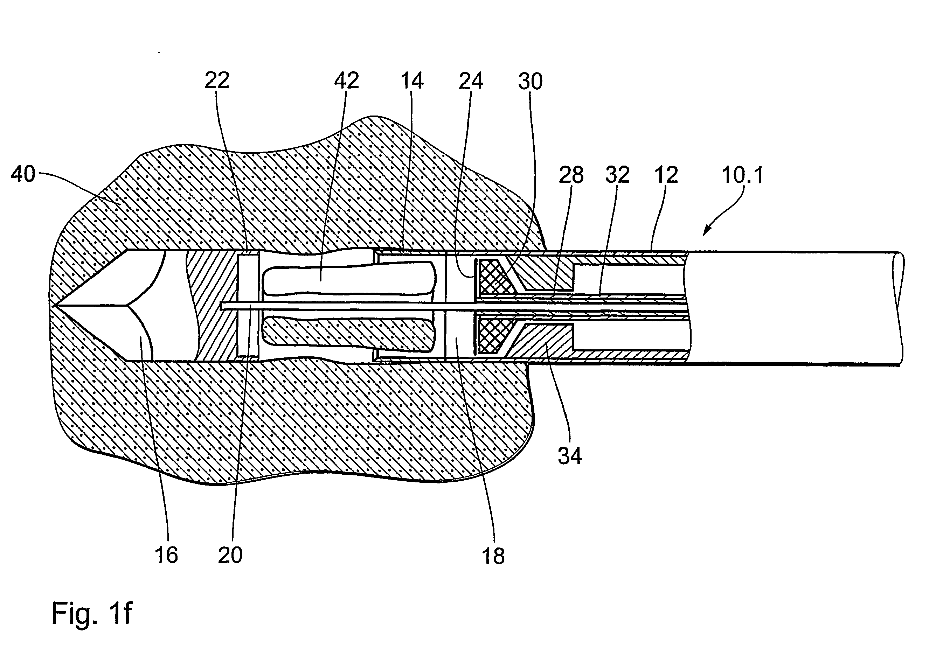

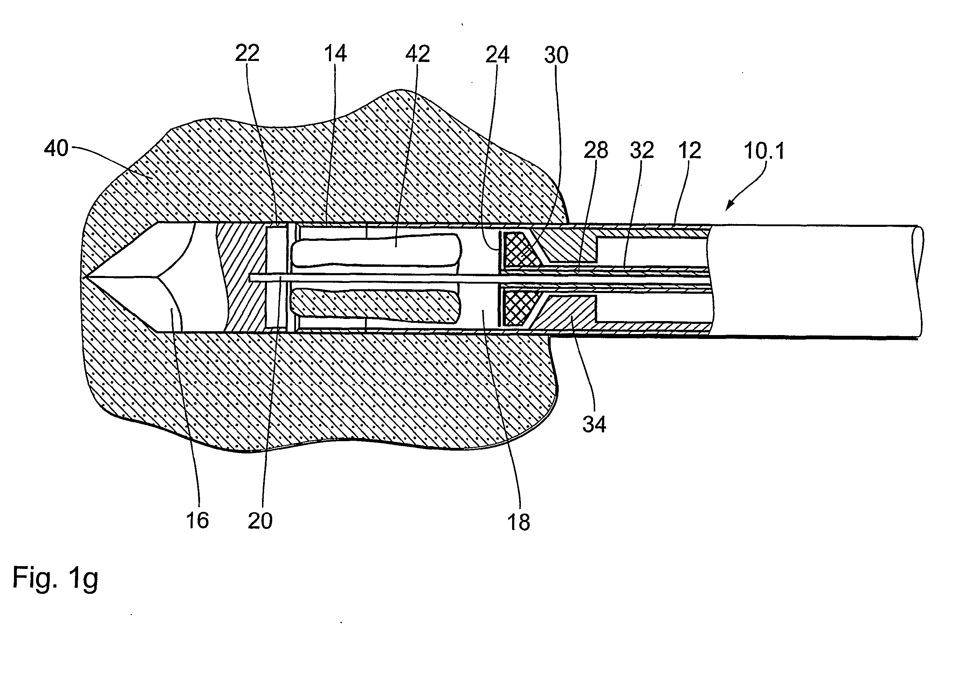

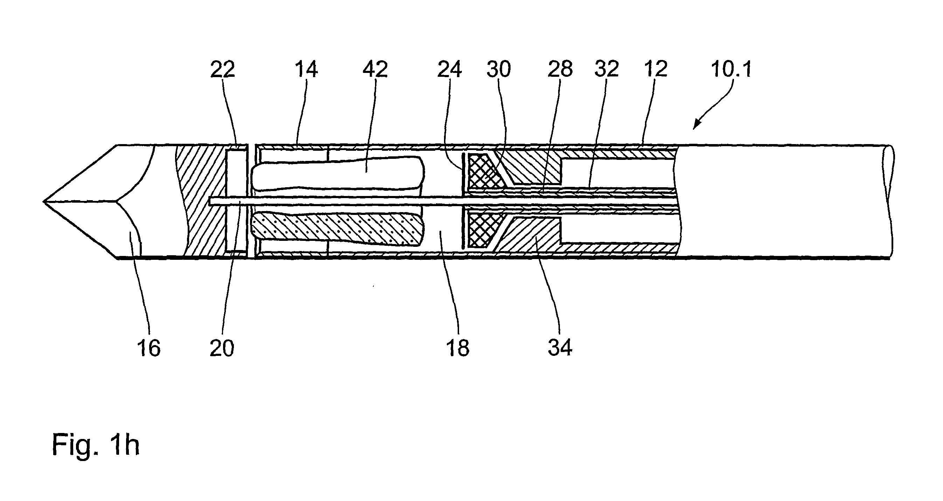

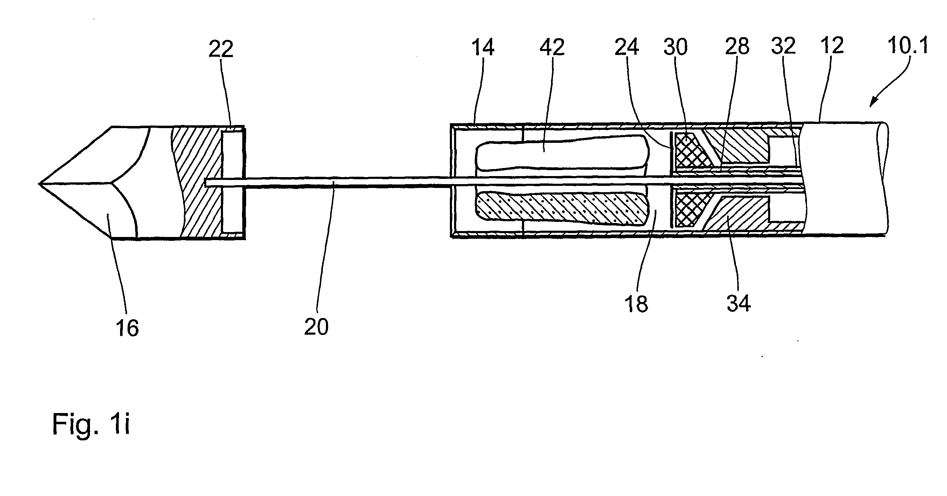

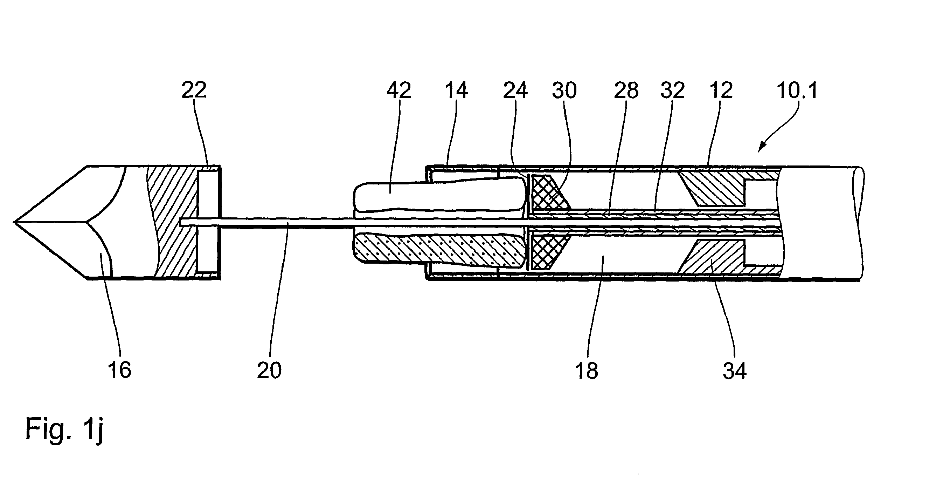

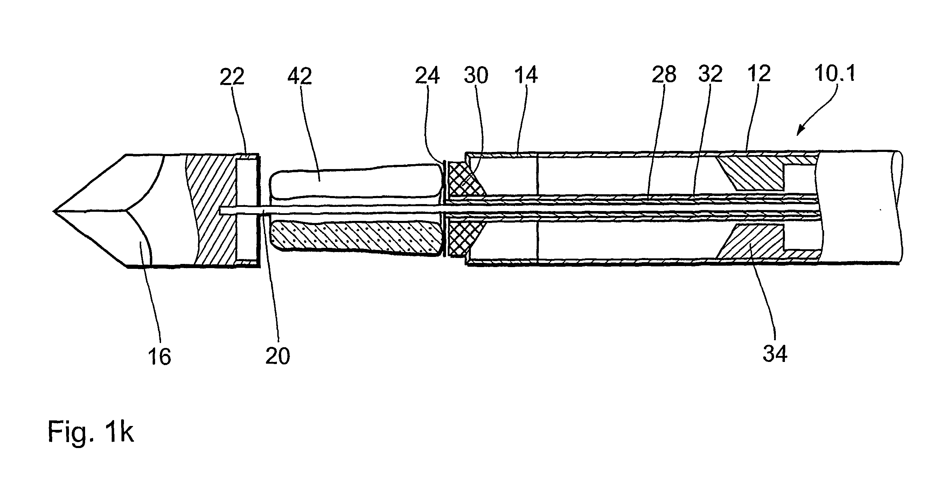

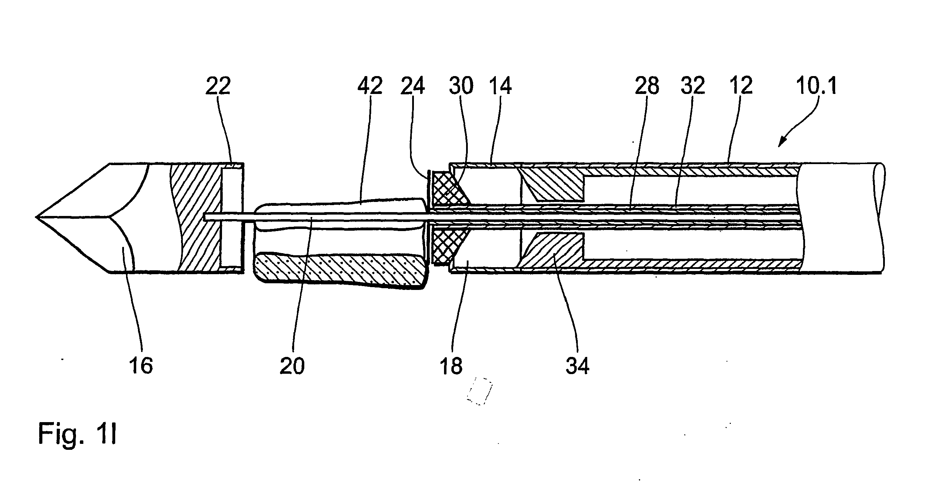

Wesentliche Bestandteile der in den Figuren 1 a bis 11 abgebildeten Hohlsonde 10.1 sind eine Metallhülse 12, welche an ihrem distalen Endabschnitt 14 isoliert ist sowie eine Metallspitze 16. Die Metallhülse 12 mit ihrem distalen Endabschnitt 14 schließt zusammen mit der Metallspitze 16 einen Hohl- oder Aufnahmeraum 18 ein. Die Metallspitze 16 ist über eine axial in der Metallhülse 1 2 verschiebbar gelagerte Schubstange 20 mit der Metallhülse 12 verbunden. Wie in den Figuren 1 b bis 1l dargestellt, können die Metallspitze 16 und die Metallhülse 12 derart relativ zueinander verschoben werden, dass sich zwischen einem Kragen 22 an der Metallspitze 16 und dem distalen Endabschnitt 14 eine zylindrische Öffnung zum Aufnahmeraum 18 wahlweise ergibt oder schließt. Die Metallhülse 12 mit ihrem distalen Endabschnitt 14 bildet auf diese Weise ein erstes Segment eines Hohlkörpers, dessen zweites Segment die relativ zum ersten Segment axial verschiebbare Metallspitze 16 ist.

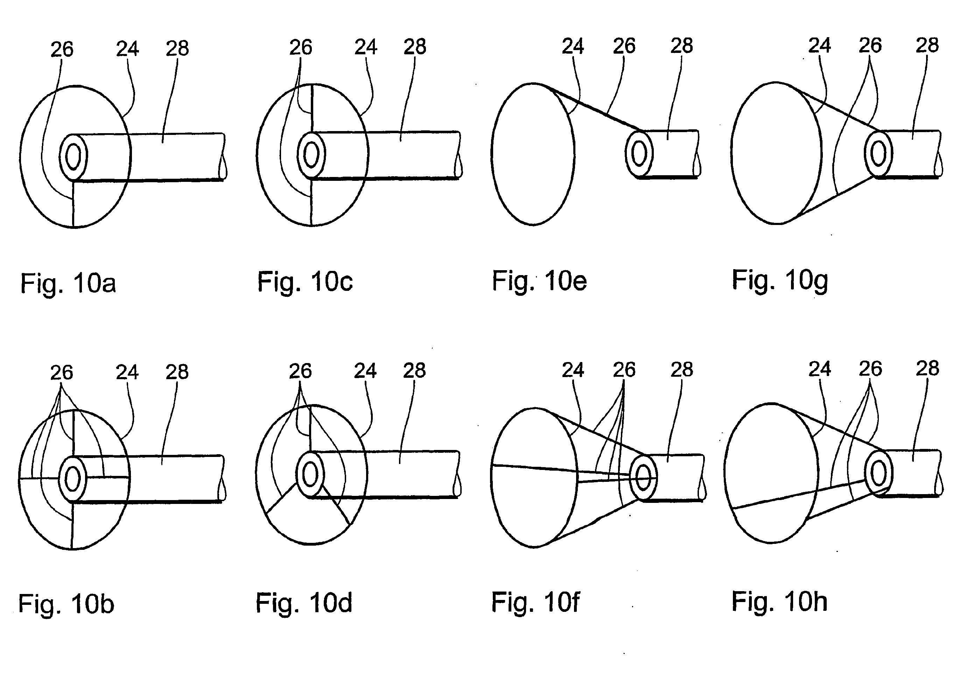

Weiterer Bestandteil der Hohlsonde 10.1 ist ein Schneidelement 24, welches von einem Drahtring gebildet wird, dessen Durchmesser geringfügig kleiner ist, als der Durchmesser des Aufnahmeraumes 18. Das Schneidelement 24 ist über eine Drahtstrebe 26 (in Figur 1 nicht abgebildet, siehe Figur 10) mit einer zweiten Schubstange 28 verbunden, die nach Art einer Schiebehülse auf der ersten Schubstange 20 für die Metallspitze 16 axial verschieblich geführt ist. Damit ist das Schneidelement 24 auch relativ zur Metallspitze 16 sowie zur Metallhülse 12 axial verschieblich.

Weiterhin ist in der Hohlsonde 10.1 ein Auswerfer 30 angeordnet, der an einer dritten Schubstange 32 befestigt ist, welcher nach Art einer Schiebehülse auf der zweiten Schubstange 28 axial verschieblich geführt ist. Der Auswerfer 30 ist nach Art eines Stempels aus der in den Figuren 1 a bis 1i abgebildeten Position in die in Figur 1 k abgebildete Position bewegbar, um im Aufnahmeraum 18 befindliches Gewebe auszuwerfen; siehe Figur 1j.

Die drei Schubstangen 20, 28 und 32 sind in einer zwischen der Metallhülse 12 und der dritten Schubstange 32 angeordneten, isolierenden Axialführung 34 geführt. Die Axialführung 34 dient gleichzeitig dazu, den Aufnahmeraum 18 gegenüber dem übrigen von der Metallhülse 12 eingeschlossenen Raum abzudichten.

Anhand der Figuren 1a bis 1l wird nun die Funktionsweise der Hohlsonde 10.1 erläutert. Zunächst wird die Hohlsonde mit der angeschärften Metallspitze 16 voran ins Gewebe eingestochen. Währenddessen wird zunächst zwischen der als erste Elektrode dienenden Metallspitze 16 und einer großflächigen Gegenelektrode am Körper des Patienten eine hochfrequente Wechselspannung angelegt. Dies hat zur Folge, dass an die Hohlsonde angrenzendes Gewebe mittels eines lokal begrenzten, hochfrequenten Wechselstroms thermisch inaktiviert wird. Wird die Hohlsonde weiter ins Gewebe eingestochen, so dass auch die Metallhülse 12 in das Gewebe eindringt, kann die Metallhülse 12 als zweite Elektrode für das Anlegen der hochfrequenten Wechselspannung dienen. Die Metallspitze 16 und die Metallhülse 12 sind dabei durch den isolierenden distalen Endabschnitt 14 der Metallhülse 12 voneinander elektrisch isoliert. Liegt das hochfrequente Wechselfeld bipolar zwischen der Metallspitze 16 und der Metallhülse 12 an, kann das Wechselfeld beim Einstechen der Hohlsonde lokal auf unmittelbar angrenzende Gewebezonen begrenzt werden. Die großflächige Gegenelektrode wird dann nicht mehr benötigt. Die angelegte hochfrequente Spannung wird so eingestellt, dass in dem an die Hohlsonde 10.1 angrenzenden Gewebe Temperaturen zwischen 55 Grad Celsius und 100 Grad Celsius, vorzugsweise zwischen 60 Grad Celsius und 80 Grad Celsius, über einen Zeitraum von 5 bis 20 Minuten erzeugt werden, um insbesondere Stoffwechselvorgänge innerhalb von Tumorzellen irreversibel zu beenden. Durch eine derartige thermische Inaktivierung wird sichergestellt, dass insbesondere Tumorzellen bei der anschließenden Gewebeentnahme nicht mehr verschleppt werden, so dass die Gefahr von Metastasen verringert wird.

Figur 1a zeigt die vollständig ins Gewebe eingestochene Hohlsonde 10.1. Nach dem Einstechen der Hohlsonde 10.1 wird die Metallhülse 12 gegenüber der Axialführung 34, dem Auswerfer 30, dem Schneidelement 24 und der Metallhülse 16 zurückgezogen. Auf diese Weise ergibt sich zwischen dem Kragen 22 der Metallspitze 16 und dem distalen Endabschnitt 14 der Metallhülse 12 eine zylinderförmige Öffnung. Das beim Einstechen der Hohlsonde 10.1 von dieser zunächst seitlich verdrängte Gewebe dringt durch die zylinderförmige Öffnung ein und legt sich an die zweite Schubstange 28 an. Dies ist in Figur 1 b abgebildet.

Anschließend wird das Schneidelement 24 mittels der zweiten Schubstange 28 in Richtung des Auswerfers 30 zurückgezogen. Währenddessen wird zwischen dem das Schneidelement 24 bildenden Drahtring und der als Gegenelektrode dienenden Metallhülse 12 eine hochfrequente Wechselspannung angelegt. Die von dem Drahtring gebildete Schneidelektrode erzeugt dabei durch die angelegte Hochfrequenzspannung viele kleine Funkenentladungen entlang des Drahtrings. Dadurch wird ein Dampfpolster zwischen dem Gewebe 40 und dem Schneidelement 24 erzeugt, welches ein berührungsfreies, elektrisches Schneiden erlaubt. Durch die Verwendung eines eher sinusförmigen hochfrequenten Wechselstroms können Schnitte im Gewebe mit minimaler thermischer Randkoagulation erzeugt werden. Andere Koagulationsgrade an der Schnittstelle können durch Änderung des sogenannten Crest-Faktors eingestellt werden, welcher das Verhältnis von Spitzenspannung zur Effektivspannung beschreibt, sowie durch zusätzliche Hochfrequenz-Modulation. Das Ausschneiden des Gewebes ist in den Figuren 1c und 1 d dargestellt, wobei insbesondere der Figur 1 d zu entnehmen ist, dass der entstehende Gewebeabschnitt 42 die Form eines die erste Schubstange 20 umgebenden Torus hat, welche aufgrund der Drahtstrebe oder Streben 26 (siehe Figur 10) derart geschlitzt ist, dass er die Schubstange 20 zwar vollständig umschließt, jedoch derart geteilt ist, dass der Gewebeabschnitt 42 später von der Schubstange 20 entfernt werden kann.

Nachdem der Gewebeabschnitt 42, wie in Figur 1e dargestellt, vollständig abgetrennt ist, befindet sich das Schneidelement 24 unmittelbar vor dem Auswerfer 30. Nach dem vollständigen Abtrennen des Gewebeabschnitts 42 wird die Metallhülse 12 wieder in Richtung der Metallspitze 16 verschoben, wie in Figur 1f abgebildet ist. Wenn die Hohlsonde 10.1, wie in Figur 1g abgebildet, anschließend geschlossen ist, wird sie wieder aus dem Gewebe 40 gezogen; Figur 1h zeigt die aus dem Gewebe 40 gezogene, geschlossene Hohlsonde 10.1. Anschließend wird die Metallspitze 16 mittels der Schubstange 20 nach vorne, bezüglich der Hohlsonde 10.1 in distaler Richtung, geschoben. Es bildet sich wieder eine zylindrische Öffnung zwischen dem Kragen 22 und dem distalen Endabschnitt 14.

Anschließend werden der Auswerfer 30 zusammen mit dem Schneidelement 24 nach vorne in Richtung der Metallspitze 16 geschoben. Dadurch wird der Gewebeabschnitt 42 aus dem Hohlraum 18 ausgeworfen, siehe Figuren 1j und 1k.

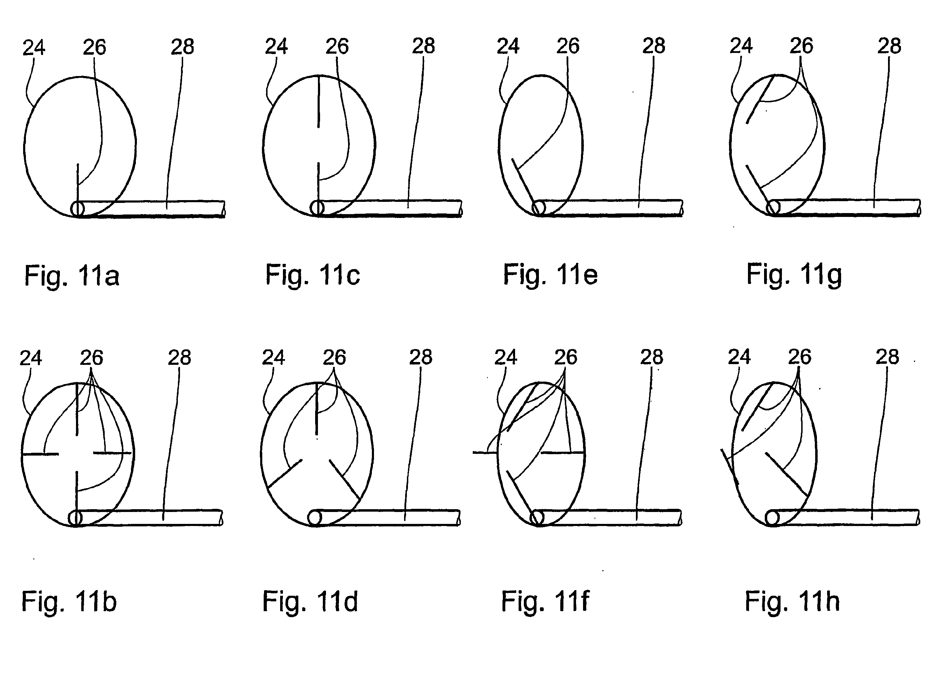

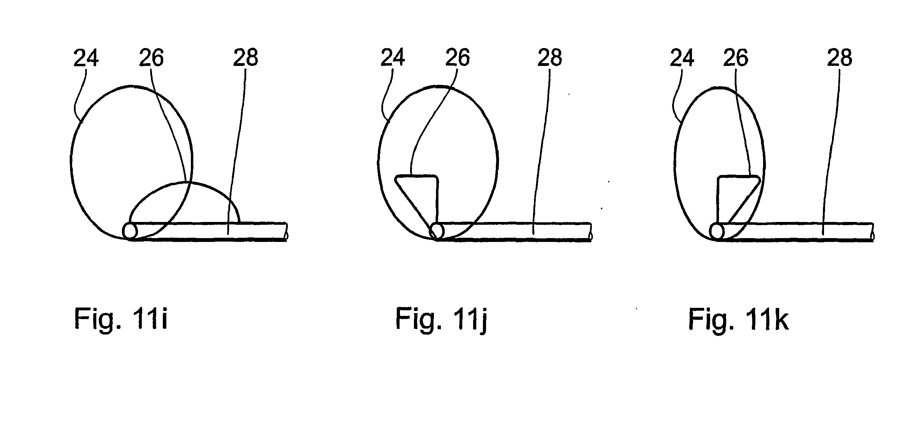

Da der Gewebeabschnitt 42 aufgrund der Drahtstrebe 26 des Schneidelementes 24 bereits geschlitzt ist, fällt er von der Schubstange 20 leicht ab, wie in Figur 11 dargestellt. Nachdem der Gewebeabschnitt 42 ausgeworfen wurde, kann die Hohlsonde 10.1 wieder in ihren in Figur 1a dargestellten Zustand versetzt werden und ist bereit für einen neuen Einsatz.

Anstelle wie beschrieben einen Gewebetorus über die volle Länge der Öffnung zwischen der Metallspitze 16 und der Metallhülse 14 auszuschneiden, indem das Schneidelement 24 entsprechend über diese Länge verfahren wird, kann auch nur ein Teil des in die Öffnung hineindringenden Gewebes abgetrennt werden. Dies geschieht, indem zunächst ein Teil des Gewebes wie bisher beschrieben geschnitten wird, so dass sich beispielsweise der in Figur 1c dargestellte Zustand einstellt. In diesem Zustand ist das bereits teilweise abgetrennte Gewebe auf der der Metallhülse 12 zugewandten Seite noch mit dem übrigen Gewebe verbunden. Wenn das Schneidelement 24 in der in Figur 1c dargestellten Stellung samt der Schubstange 28 um deren Längsachse gedreht wird, schneiden der oder die Stege oder Streben 26 (siehe Figur 10) das bis dahin teilweise abgetrennte Gewebe ganz vom übrigen Gewebe ab. Durch Schließen der Öffnung zwischen der Metallspitze 16 und der Metallhülse 12 kann dann auch ein kleiner Gewebeabschnitt entnommen werden. Dies lässt sich auch dadurch erreichen, dass die Öffnung zwischen der Metallspitze 16 und der Metallhülse 12 nicht ganz so weit geöffnet wird, wie in den Figuren 1b bis 1e dargestellt, sondern nur teilweise, so dass insgesamt auch weniger Gewebe in die dann kleinere Öffnung zwischen der Metallspitze 16 und der Metallhülse 12 eindringt.

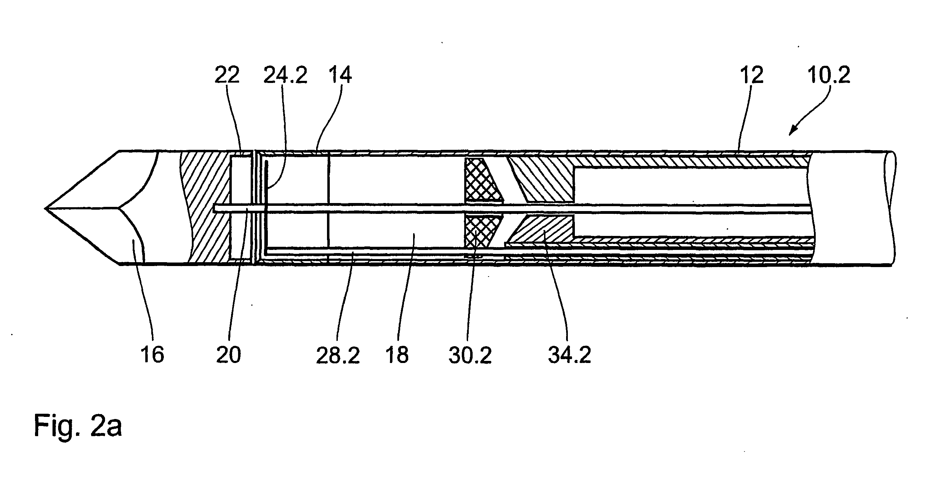

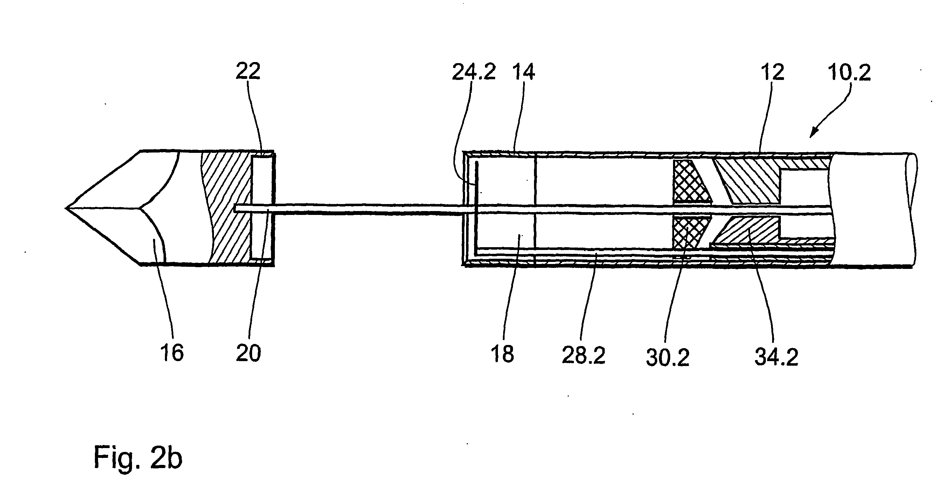

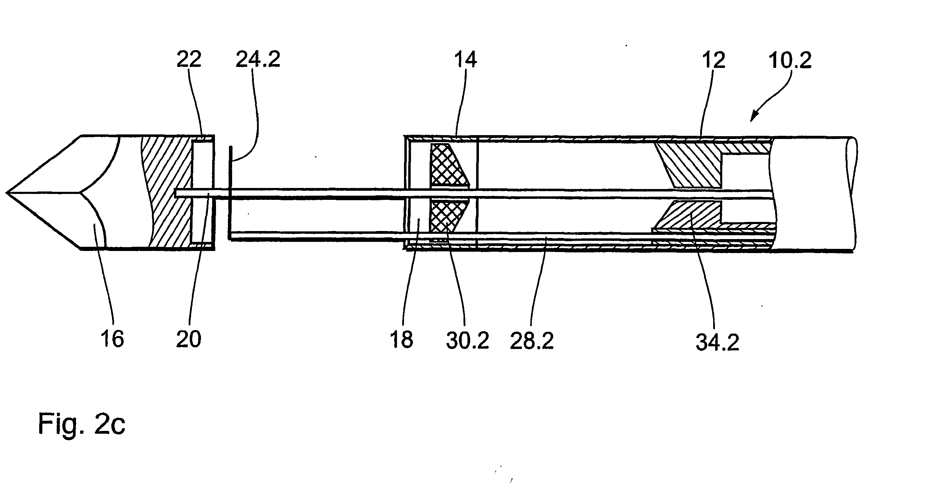

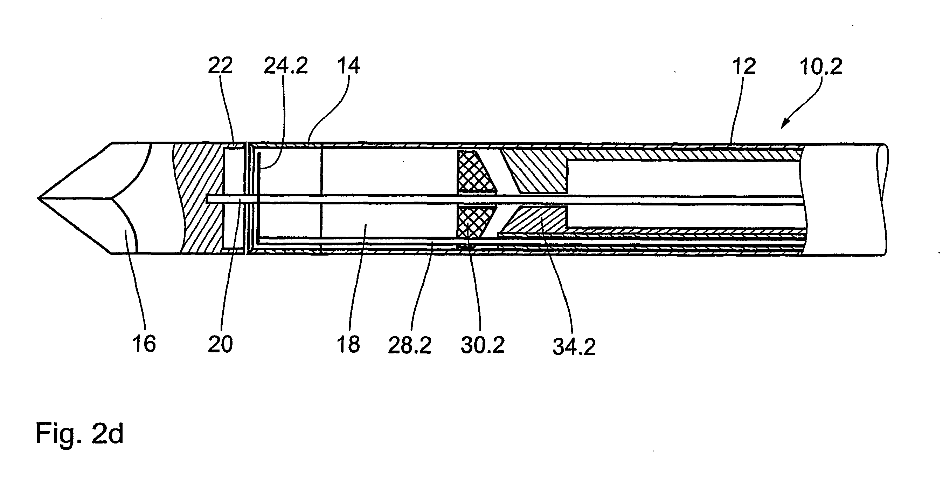

Die in den Figuren 2a bis 2e dargestellte Hohlsonde 10.2 unterscheidet sich von der in den Figuren 1a bis 1l dargestellten Hohlsonde 10.1 in einigen konstruktiven Details. Im Folgenden werden nur die Unterschiede der Hohlsonde 10.2 gegenüber der in Figur 1 dargestellten Hohlsonde 10.1 dargestellt. Die Schubstange 28.2 für das Schneidelement 24.2 ist nicht zentral, sondern an der Seite des Aufnahmeraumes 18 angeordnet. Außerdem ist keine gesonderte Schubstange für den Auswerfer 30.2 vorgesehen. Vielmehr ist der Auswerfer 30.2 ebenso wie das Schneidelement 24.2 an der Schubstange 28.2 befestigt. Das Schneidelement 24.2 und der Auswerfer 30.2 werden also gleichermaßen durch die Schubstange 28.2 bewegt und behalten ihren festen Abstand voneinander. Wie sich aus Figur 2b ergibt, wird zum Öffnen der Öffnung zwischen der Metallspitze 16 und der Metallhülse 12 die Metallhülse 12 zusammen mit der Schubstange 28.2 in dem Schneidelement 24.2 zurückgezogen, so dass sich unmittelbar nach dem Öffnen der Öffnung das Schneidelement 24.2 noch innerhalb des distalen Endabschnittes 14 der Metallhülse 12 befindet. Zum Ausschneiden von Gewebe wird dann die Schubstange 28.2 zusammen mit dem Schneidelement 24.2 und dem Auswerfer 30.2 in Richtung der Metallspitze 16 nach vorne geschoben. Dies ist in Figur 2c abgebildet. Sobald das Schneidelement 24.2 seine Endposition in der Nähe der Metallspitze 16 erreicht hat und ein entsprechender Gewebeabschnitt abgetrennt ist, wird die Metallhülse 12 relativ zur Metallspitze 16 und deren Schubstange 20 sowie relativ zum Schneidelement 24.2, dessen Schubstange 28.2 und dem daran befestigten Auswerfer 30.2 in Richtung der Metallspitze 16 geschoben um die Öffnung zu schließen. Der geschlossene Zustand der Hohlsonde 10.2 ist in Figur 2d abgebildet.

Die geschlossene Hohlsonde 10.2 wird anschließend zusammen mit dem abgetrennten Gewebe aus dem Gewebe herausgezogen. Nach dem Herausziehen der Hohlsonde 10.2 aus dem Gewebe wird diese zum Auswerfen des abgetrennten Gewebes wieder geöffnet. Dazu wird Metallhülse 12 relativ zur Metallspitze 16 und dem Schneidelement 24.2 sowie dem Auswerfer 30.2 zurückgezogen, das heißt, die Metallspitze 16, das Schneidelement 24.2 und der Auswerfer 30.2 behalten ihre relative Position zueinander. Aufgrund des Auswerfers 30.2 wird das Gewebe nicht zusammen mit der Metallhülse 12 zurückgezogen, sondern aus dem Aufnahmeraum 18 ausgeworfen.

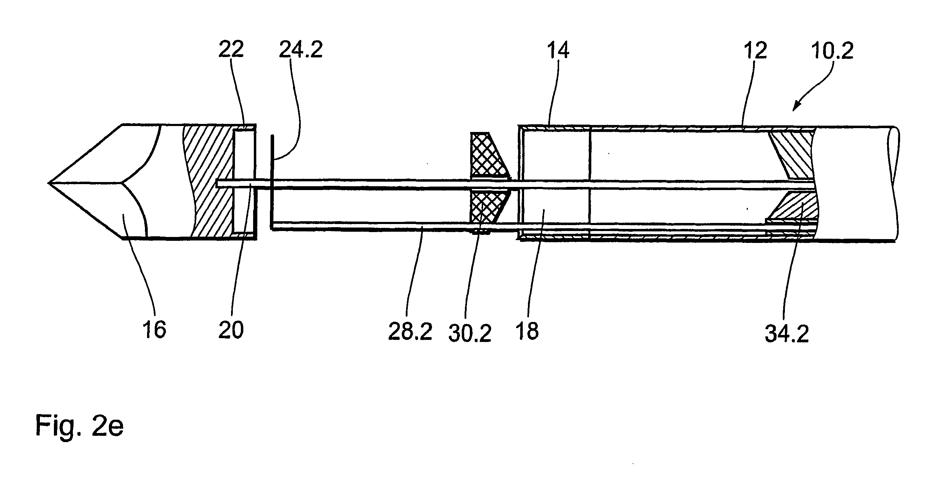

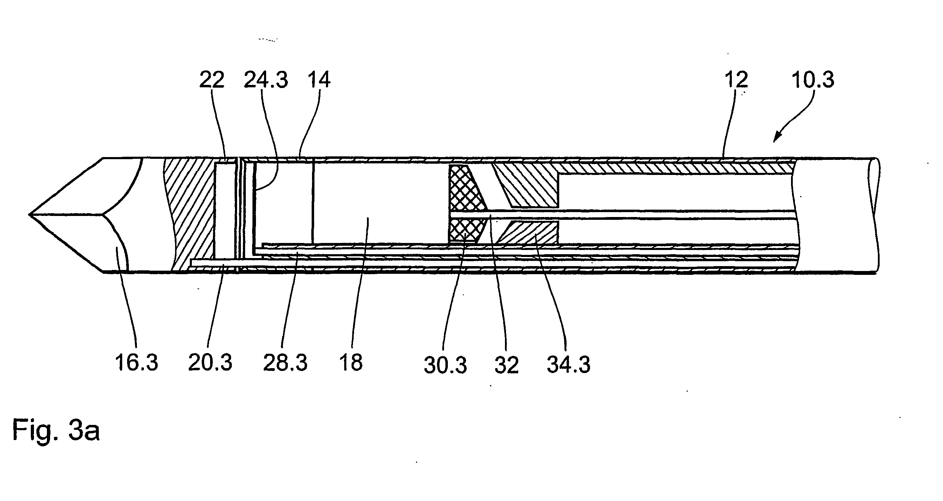

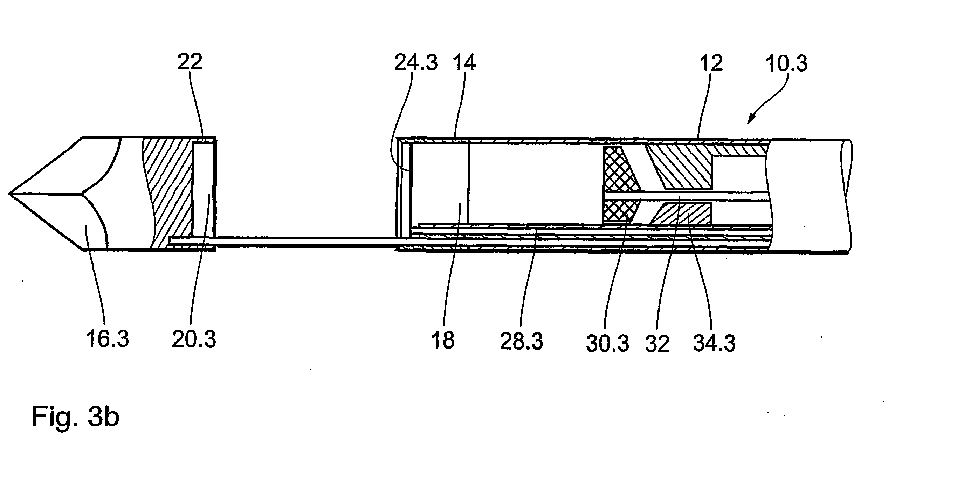

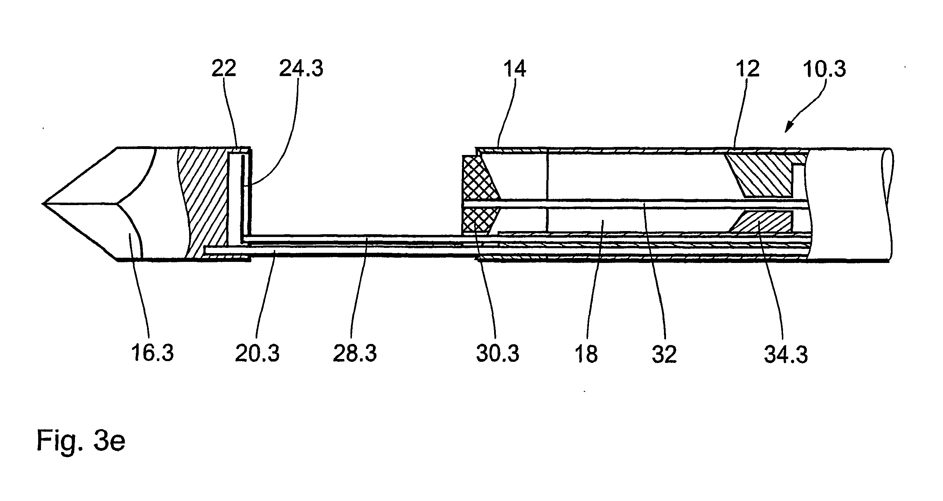

Bei der in den Figuren 3a bis 3e abgebildeten Hohlsonde 10.3 sind sowohl für die Metallspitze 16.3 als auch für das Schneidelement 24.3 seitlich des Aufnahmeraums angeordneten Schubstangen 20.3 bzw. 28.3 vorgesehen. Der Auswerfer 30.3 besitzt wie in Figur 1 eine zentrale Schubstange 32. Mittels der Schubstangen 20.3, 28.3 und 32 lassen sich die Metallspitze 16.3, das Schneidelement 24.3 und der Auswerfer 30.3 wie bei der Hohlsonde 10.1 aus Figur 1 separat bewegen. Der in den Figuren 3b bis 3e dargestellte Bewegungsablauf unterscheidet sich von dem in Figur 1 dargestellten Bewegungsablauf dadurch, dass die Metallhülse 12 zusammen mit dem Schneidelement 24.3 und dem Auswerfer 30 zum Öffnen der Öffnung zwischen der Metallhülse 12 und der Metallspitze 16 zurückgezogen wird, nachdem die Hohlsonde 10.3 in Gewebe eingestochen wurde. Zum Ausschneiden eines Gewebeabschnittes wird dann das Schneidelement 24.3 wie bei der in Figur 2 dargestellten Variante aus der Metallhülse 12 heraus nach vom in Richtung der Metallspitze 16 geschoben. Anschließend wird die Hohlsonde 10.3 geschlossen und samt des abgetrennten Gewebeabschnitts dem Gewebe entnommen. Das Auswerfen des abgetrennten Gewebeabschnittes erfolgt nach Öffnen der Hohlsonde 10.3 mithilfe des Auswerfers 30.3 in gleicher Weise wie in Figur 1 abgebildet; siehe Figur 3e.

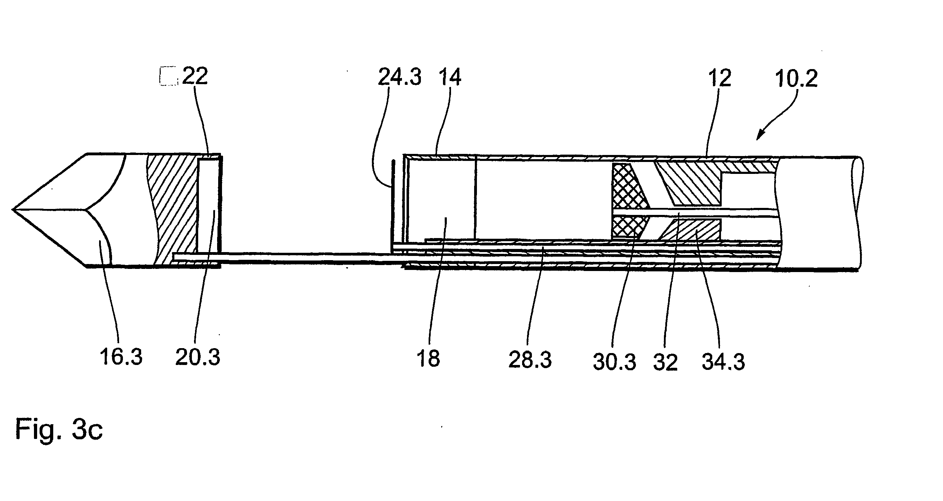

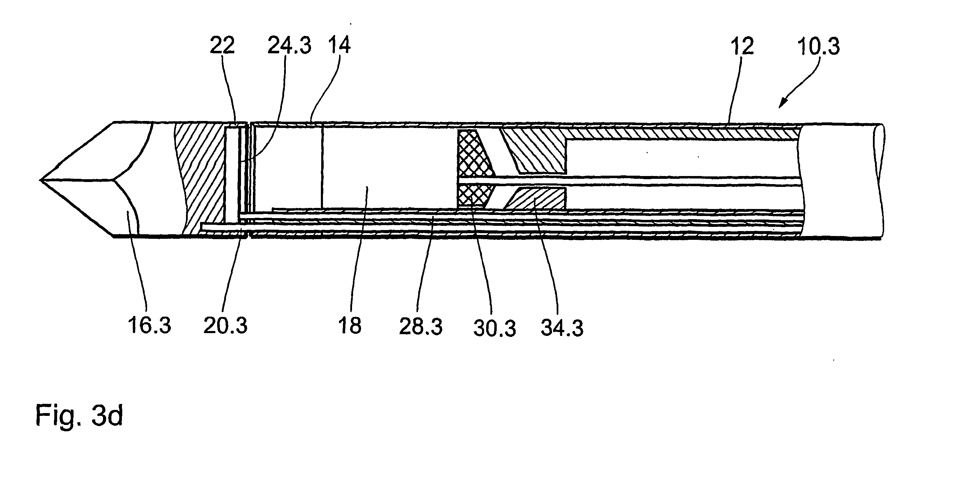

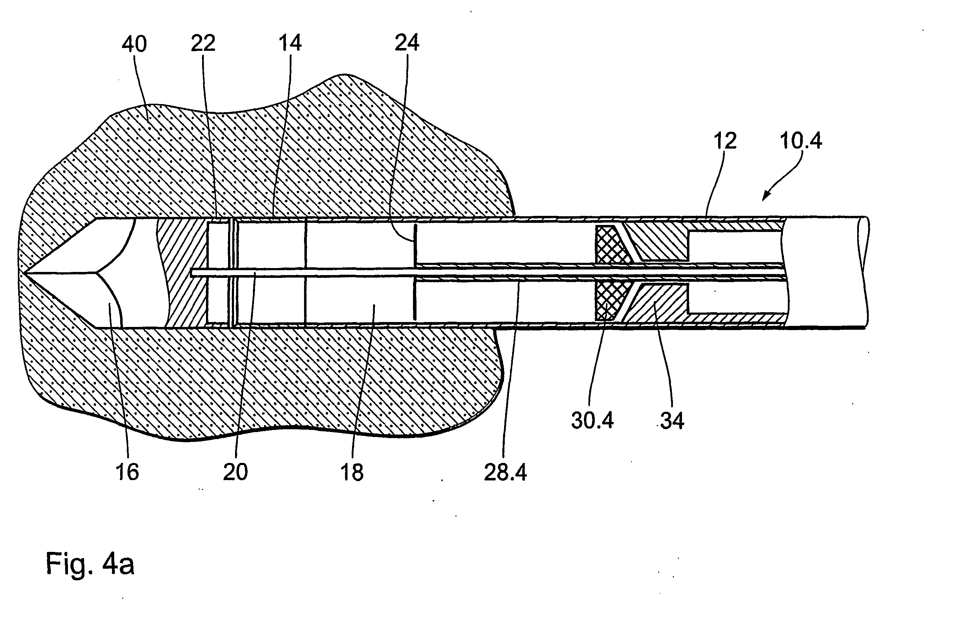

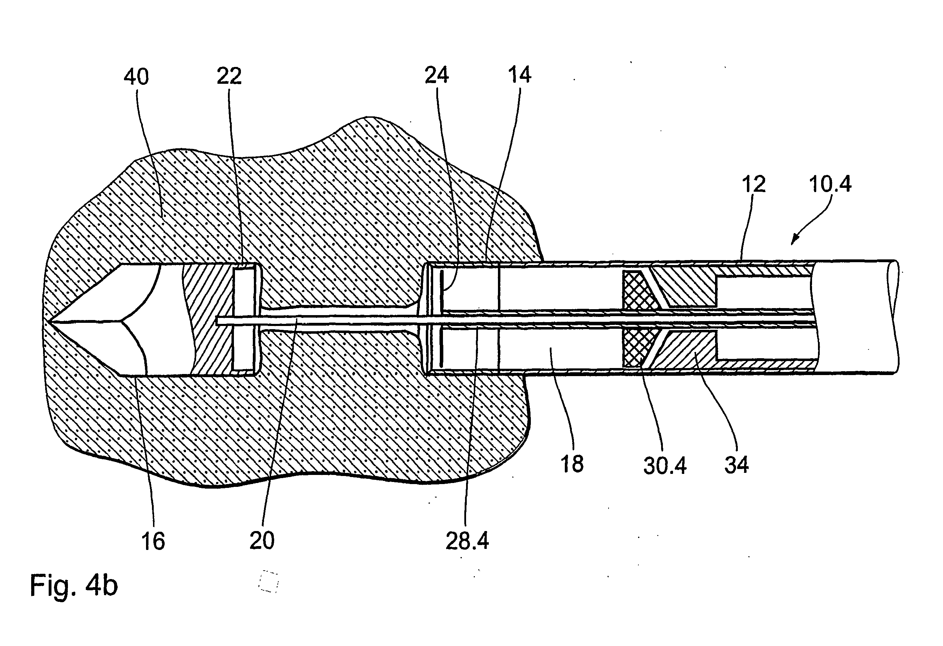

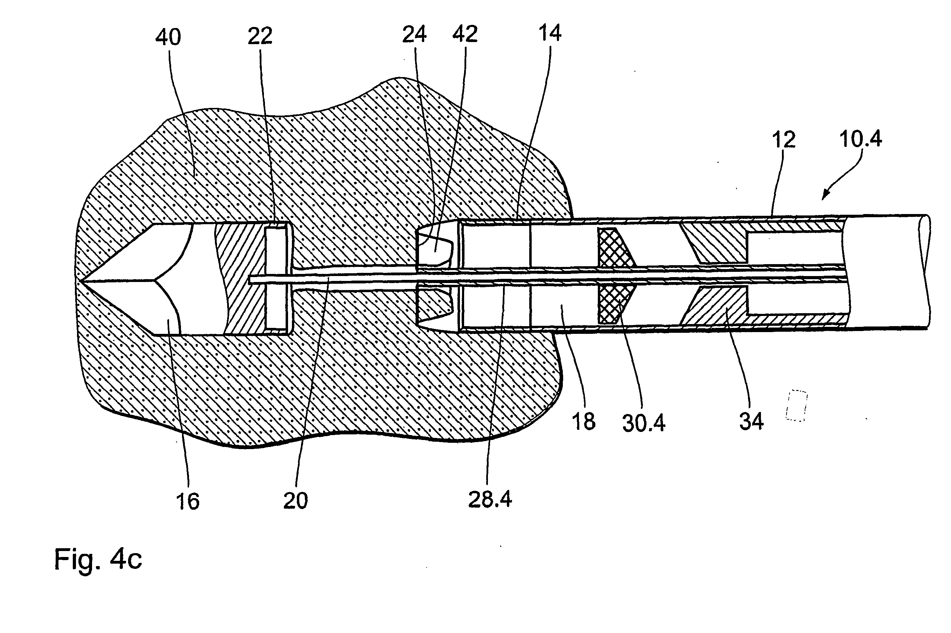

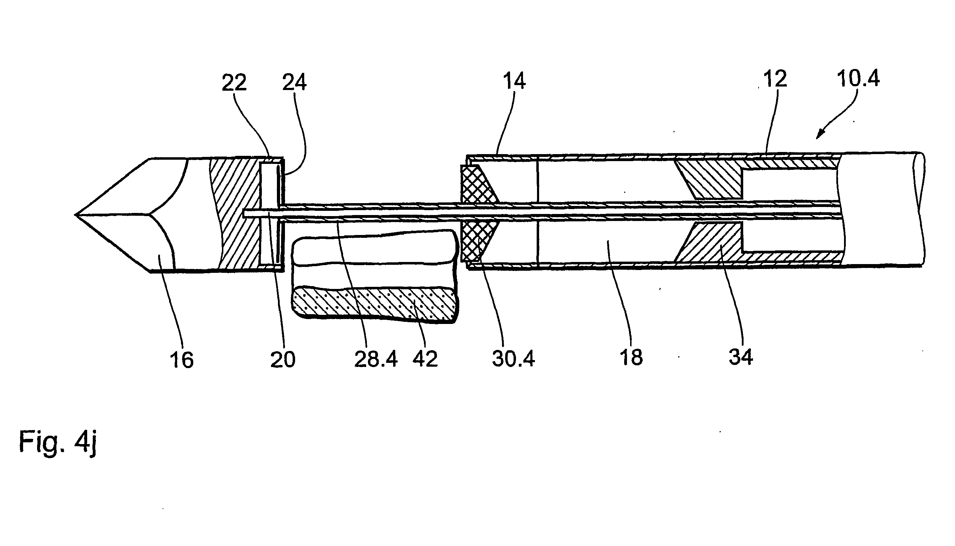

Die in den Figuren 4a bis 4j dargestellte Variante einer Hohlsonde 10.4 besitzt abweichend von der Hohlsonde aus Figur 1 eine zentrale Schubstange 28.4, an deren distalem Ende das Schneidelement 24 befestigt ist und an der außerdem ein Auswerfer 30.4 befestigt. Wie bei der in Figur 2 dargestellten Variante werden somit das Schneidelement 24 und der Auswerfer 30.4 durch eine gemeinsame Schubstange 28.4 bewegt. Die Bewegung des Schneidelementes 24 erfolgt dabei nach Öffnen der Hohlsonde 10.4 wie bei der in Figur 3 dargestellten Variante aus der Metallhülse 12 heraus zur Metallspitze 16 hin. Wie in den Figuren 4c bis 4e dargestellt, wird der Auswerfer 30.4 gleichzeitig mit dem Schneidelement 24 in Richtung auf die Metallspitze 16 zu geschoben.

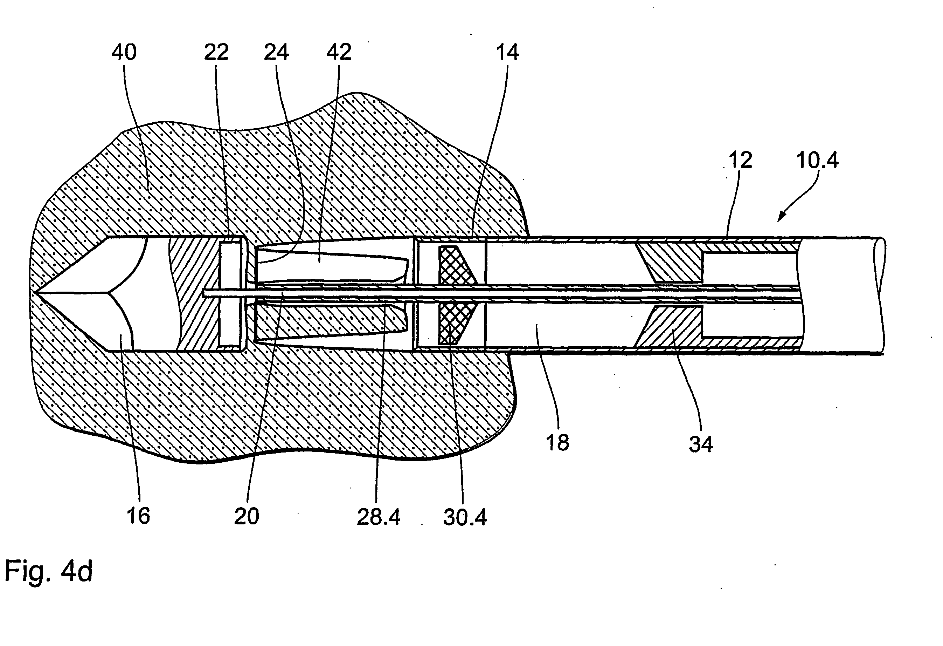

Wenn das Schneidelement 24 in seiner in Figur 4c oder Figur 4d dargestellten Position um die Längsachse seiner Schubstange 28.4 gedreht wird, schneidet die das ringförmige Schneidelement 24 mit der Schubstange 28.4 verbindende Strebe 26 den bereits teilweise abgetrennten Gewebeabschnitt 42 vom übrigen Gewebe 40 vollständig ab.

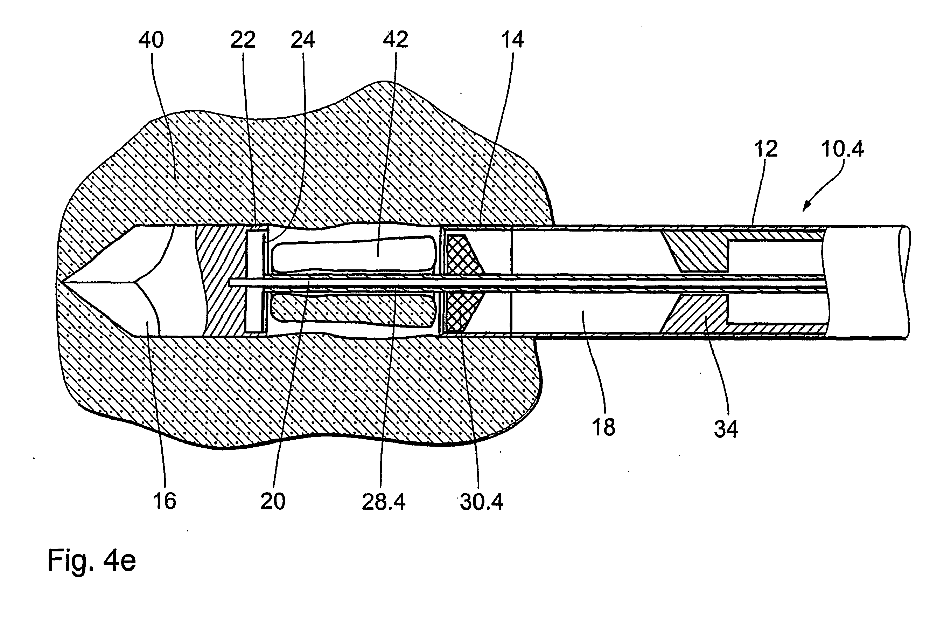

Nach dem vollständigen Abtrennen eines Gewebeabschnittes 42 - wie in Figur 4e dargestellt - wird die Hohlsonde 10.4 wieder geschlossen. Dazu kann entweder die Metallhülse 12 nach vorne in Richtung der Metallspitze 16 geschoben werden, es kann aber auch die Metallspitze 16 zusammen mit dem Schneidelement 24 und dem Auswerfer 30 in den Aufnahmeraum 18 zurückgezogen werden. In einer bevorzugten Ausführungsform schließt der Auswerfer 30 dichtend mit der Wandung der Metallhülse 12 ab. Dies unterstützt ein Einsaugen eines abgetrennten Gewebeabschnittes 42 in den Aufnahmeraum 18, während die Hohlsonde 10.4 geschlossen wird.

Neben einer Art Vakuum, die durch das Bewegen des Auswerfers 30 in dem Aufnahmeraum 18 erzeugt wird, kann an den Aufnahmeraum 18 auch zusätzlich über externe Mittel Vakuum angelegt werden. In diesem Fall ist es bevorzugt, wenn der Auswerfer 30 nicht dichtend mit der Wandung der Metallhülse 12 abschließt. Das Anlegen eines Vakuums an den Aufnahmeraum 18 kann auch bei allen übrigen Erfindungsvarianten vorgesehen sein.

Nach dem Schließen der Hohlsonde 10.4 wird diese dem Gewebe 40 entnommen. Das anschließende Öffnen der Hohlsonde 10.4 (Figur 4i) sowie das Auswerfen des abgetrennten Gewebeabschnittes 42 mittels des Auswerfers 30 (Figur 4j) erfolgt ähnlich wie bei den zuvor beschriebenen Erfindungsvarianten.

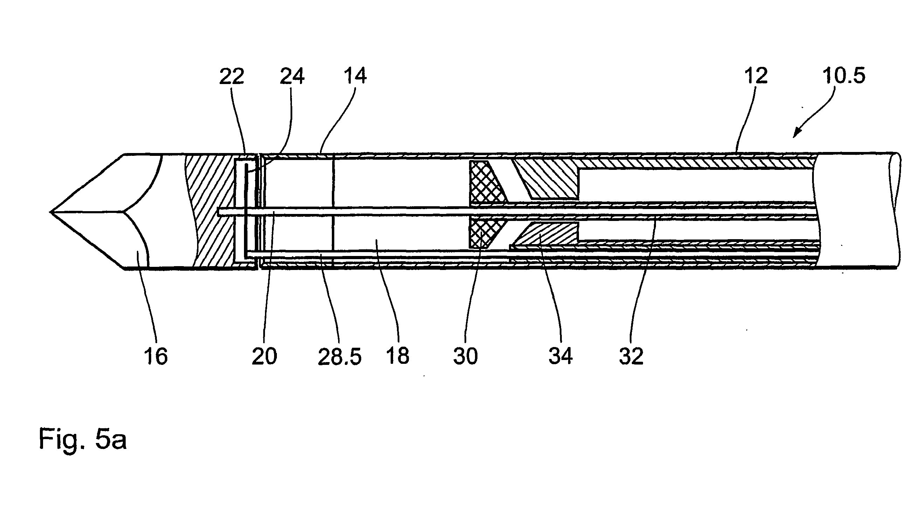

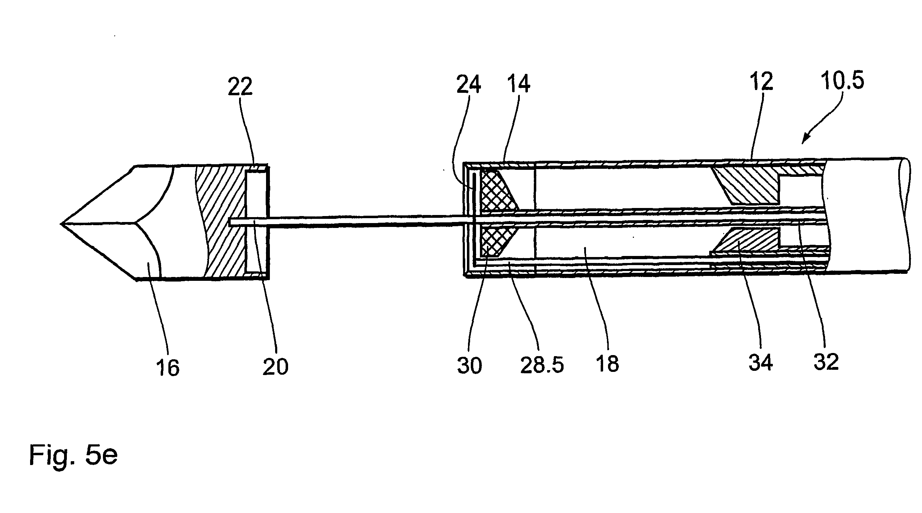

In Figur 5a ist eine Hohlsondenvariante 10.5 abgebildet. Ähnlich wie bei den in den Figuren 2 und 3 abgebildeten Varianten ist die Schubstange 28.5 seitlich des Aufnahmeraums 18 angeordnet. Wie bei der in Figur 1 beschriebenen Variante sind die Metallspitze 16 und der Auswerfer 30 durch jeweils eigene zentrale Schubstangen 20 bzw. 32 separat bewegbar. Der vorgesehene Bewegungsablauf ist ähnlich wie in Figur 1 dargestellt, mit dem Unterschied, dass der Auswerfer 30 beim Zurückziehen der Metallhülse 12 nach dem Einstechen der Hohlsonde 10.5 in entsprechendes Gewebe zum Öffnen der Öffnung der Metallspitze 16 und der Metallhülse 12 nicht mit der Metallhülse 12 zurückgezogen wird, so dass er sich nach dem Öffnen der Hohlsonde 10.5 im Bereich des distalen Endabschnitts 14 der Metallhülse 12 befindet. Das Abschneiden eines Gewebeabschnittes erfolgt durch Zurückziehen des Schneidelementes 24 von der Metallspitze 16 zum distalen Endabschnitt 14 der Metallhülse 12. Während des Schließens der Hohlsonde 10.5 wird der Auswerfer 30 zusammen mit dem Schneidelement 24 sukzessive in den Aufnahmeraum 18 zurückgezogen und kann, wenn der Auswerfer 30 dichtend an der Wandung der Metallhülse 12 anliegt, ein Einsaugen des abgetrennten Gewebeabschnittes in den Aufnahmeraum 18 unterstützen. Das Öffnen der Hohlsonde 10.5 nach ihrer Entnahme aus dem Gewebe sowie das Auswerfen abgetrennten Gewebes erfolgt analog zu den zuvor dargestellten Varianten; siehe Figur 5e.

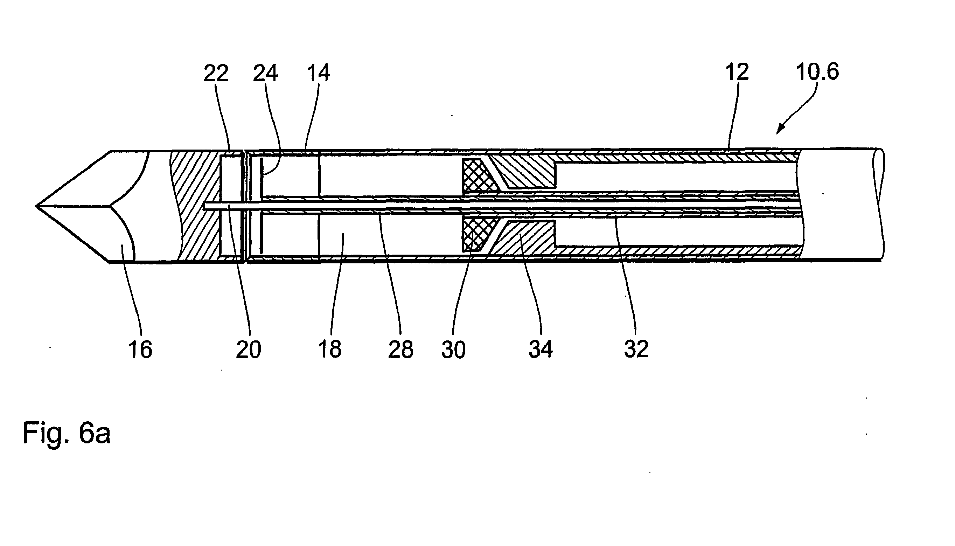

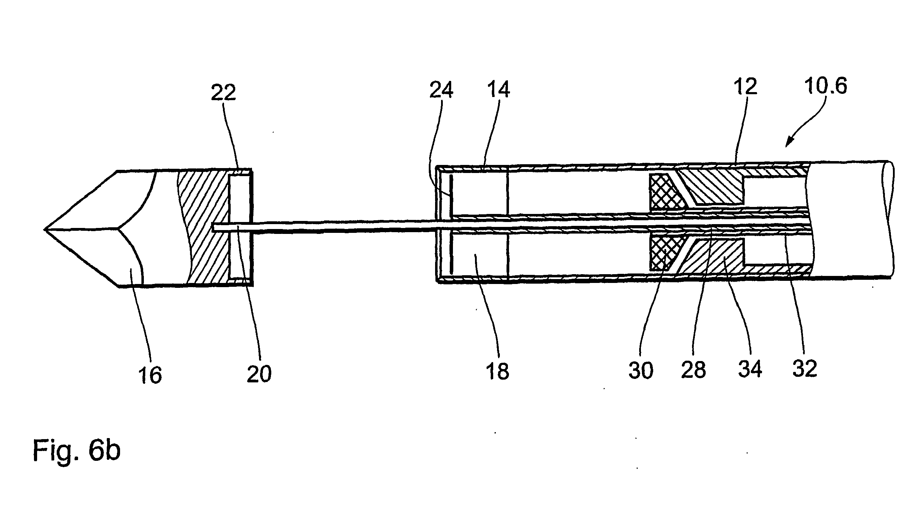

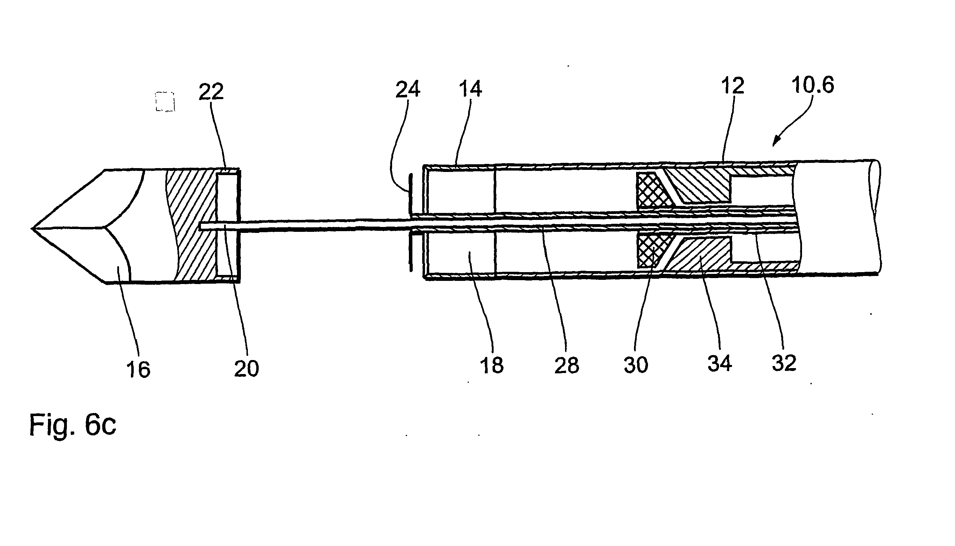

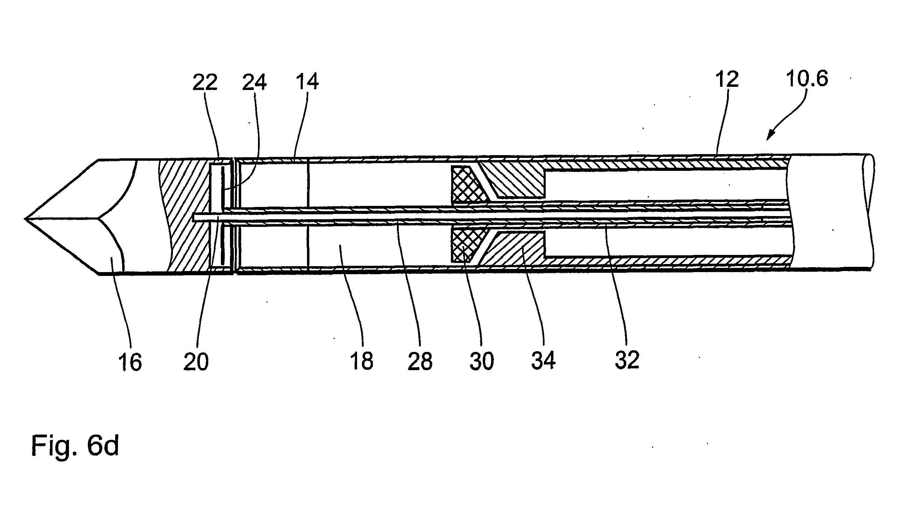

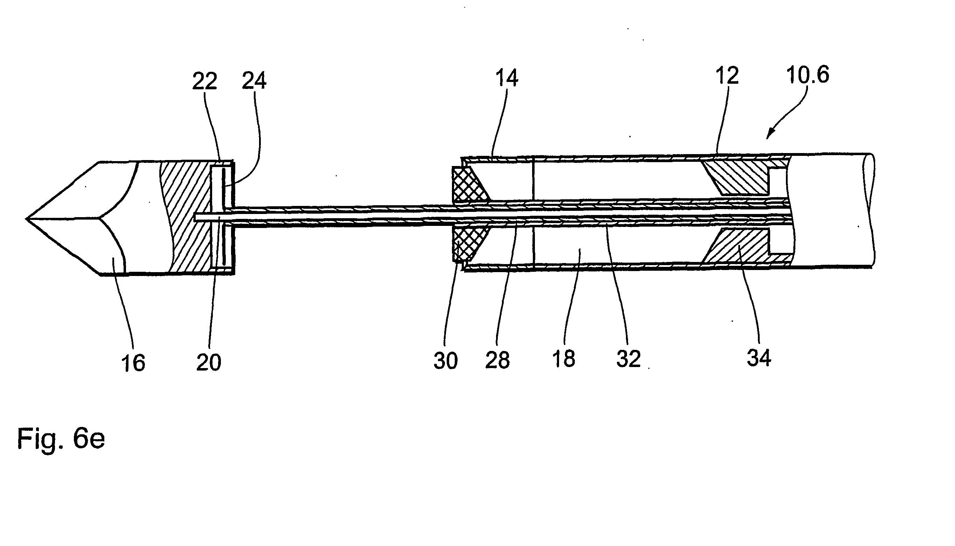

Die in den Figuren 6a bis 6e dargestellte Hohlsondenvariante 10.6 entspricht in ihrem Aufbau der in den Figuren 1a bis 1l dargestellten Variante 10.1. Damit sind die Metallspitze 16, das Schneidelement 24 und der Auswerfer 30 durch jeweils separate Schubstangen unabhängig voneinander bewegbar. Die in den Figuren 1 und 6 abgebildeten Varianten unterscheiden sich dadurch, dass das Schneidelement 24 nach Öffnen der Hohlsonde 10.6 nicht von der Metallspitze 16 ausgehend zur Metallhülse 12 zurückgezogen wird, sondern aus dem Aufnahmeraum 18 heraus in Richtung der Metallspitze 16 vorgeschoben wird, um bei geöffneter Hohlsonde 10.6 in die Öffnung zwischen Metallspitze 16 und Metallhülse 12 eindringendes Gewebe abzutrennen. Figur 6e zeigt die Stellung, in der ein abgetrennter Gewebeabschnitt ausgeworfen wird.

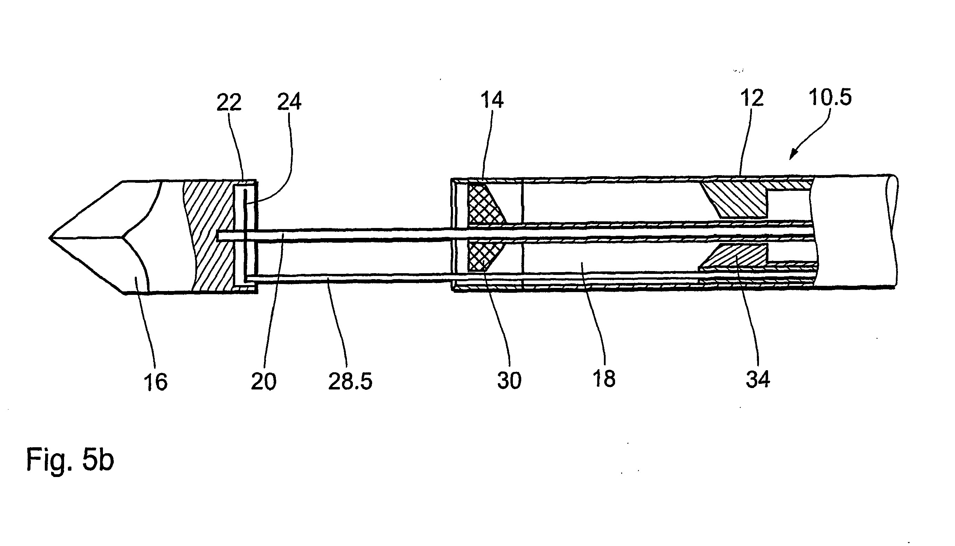

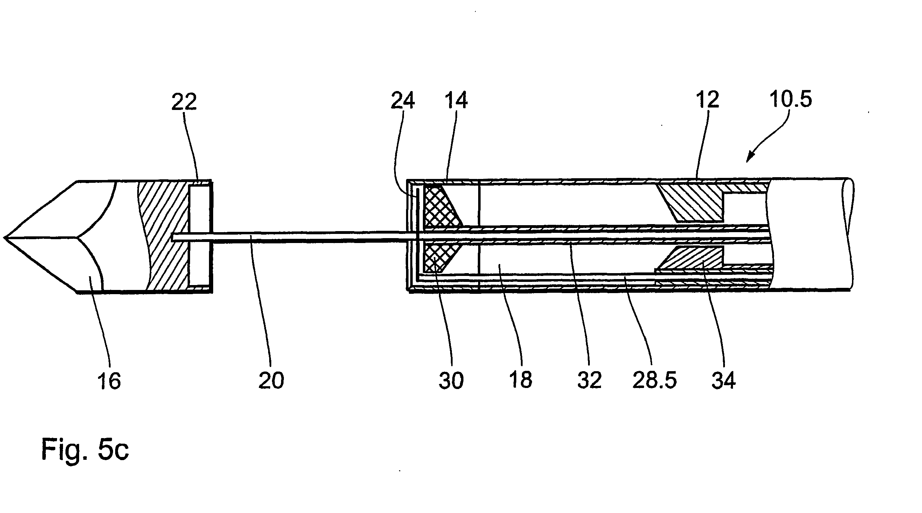

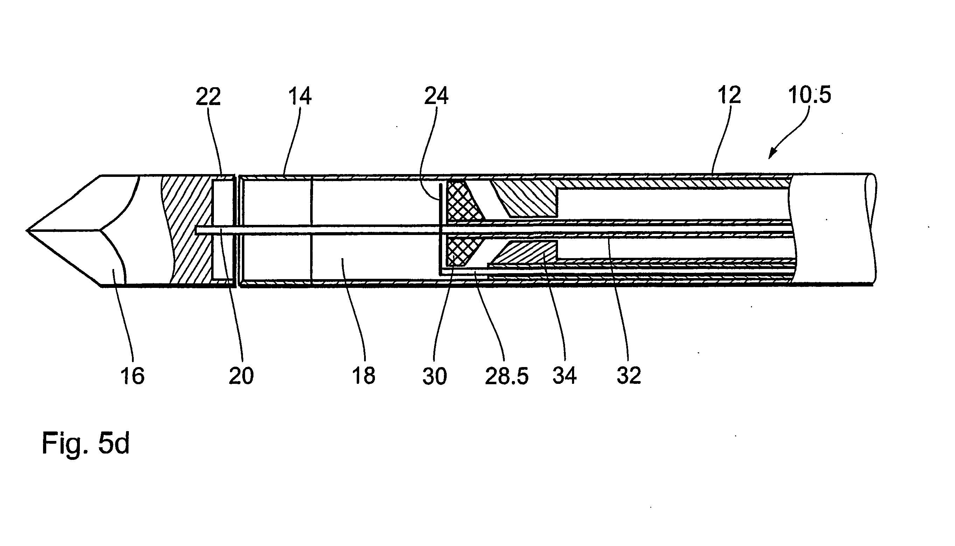

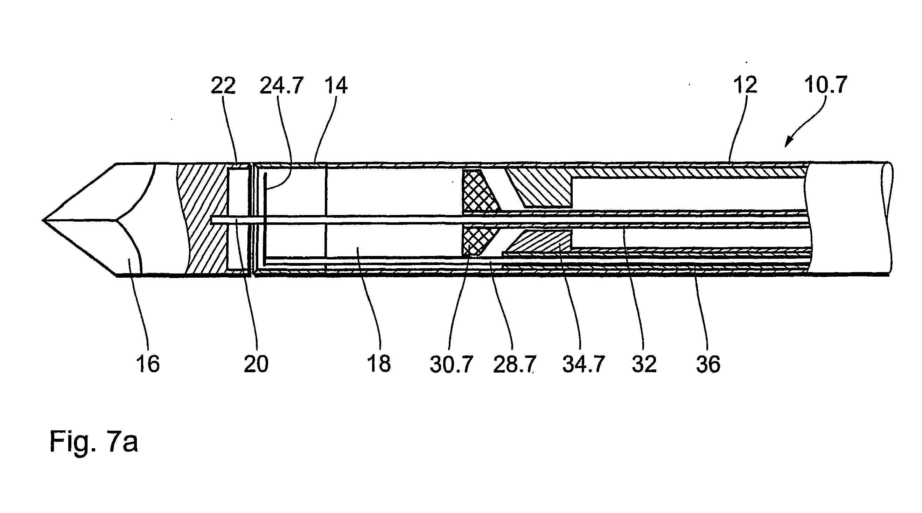

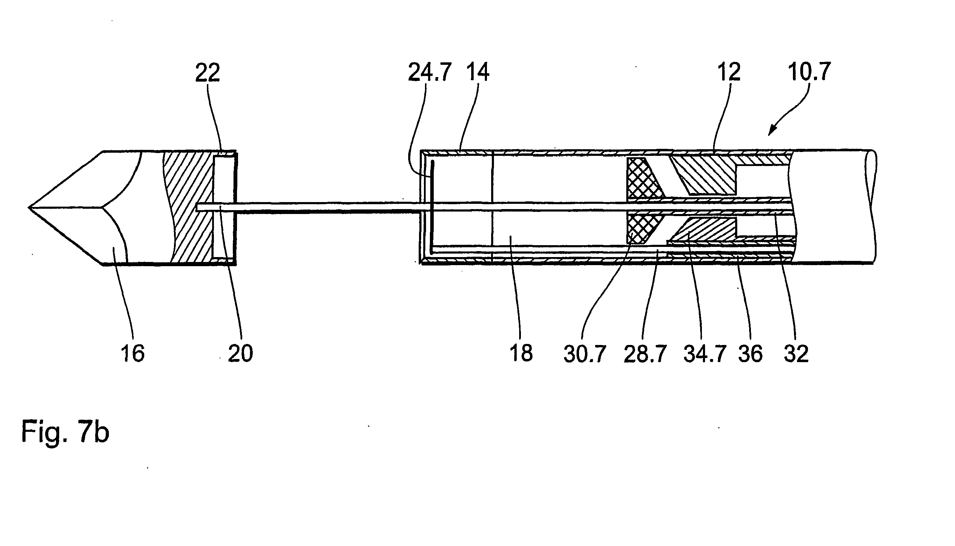

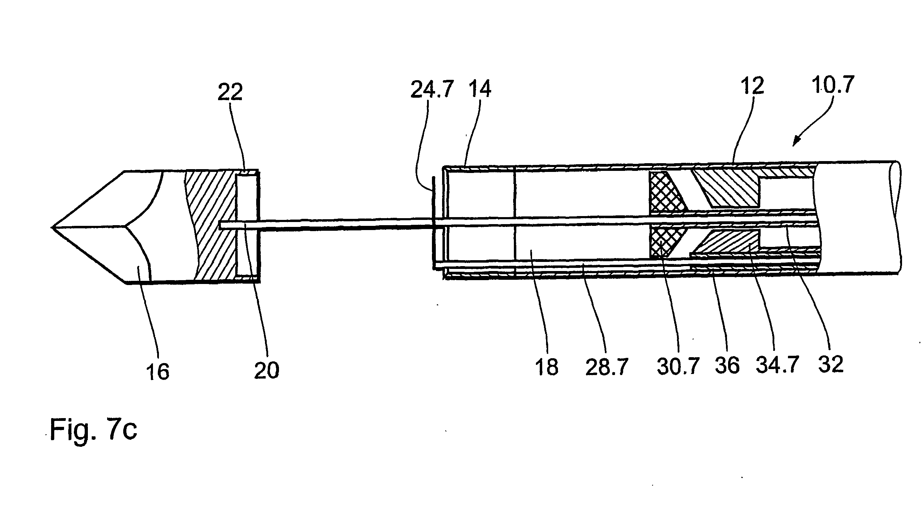

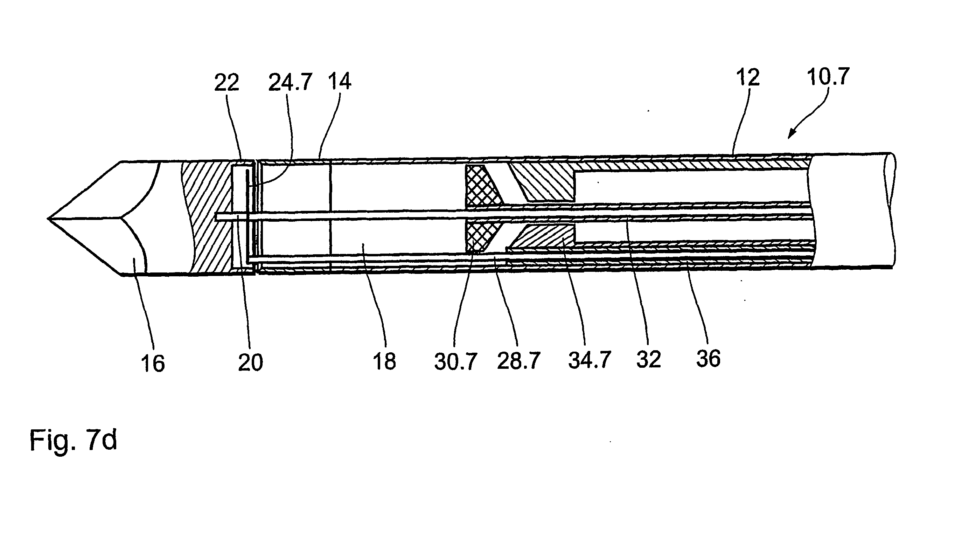

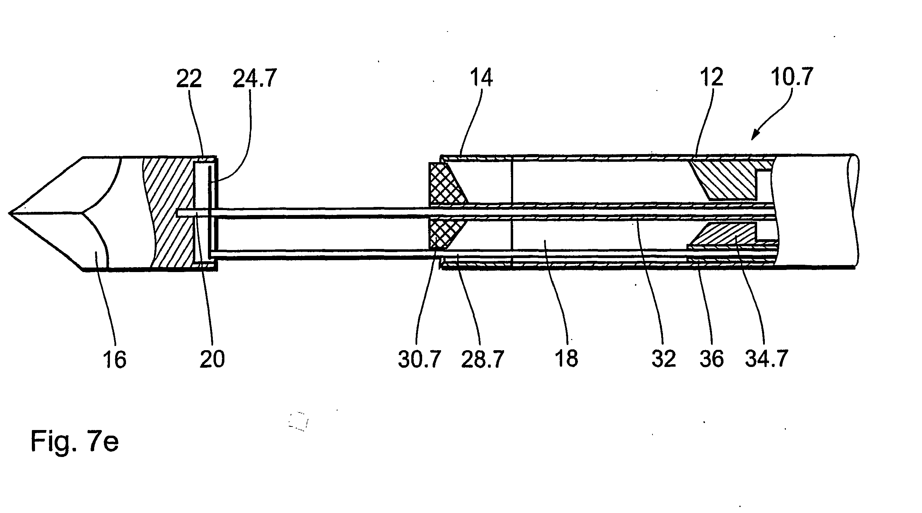

Die in den Figuren 7a bis 7e dargestellte Hohlsondenvariante 10.7 entspricht hinsichtlich ihres Aufbaus der in den Figuren 5a bis 5e dargestellten Variante 10.5. Im Unterschied zu der in Figur 5 dargestellten Variante wird das Schneidelement 24 nach dem Öffnen der Hohlsonde nicht von der Metallspitze 16 ausgehend in den distalen Endabschnitt 14 der Metallhülse 12 zurückgezogen, sondern aus dem distalen Endabschnitt 14 heraus auf die Metallspitze 16 zugeschoben. Der Bewegungsablauf entspricht somit der in den Figuren 6a bis 6e dargestellten Variante. Die dezentrale Schubstange 28.7 für das Schneidelement 24 ist in einer Führungshülse 36 geführt.

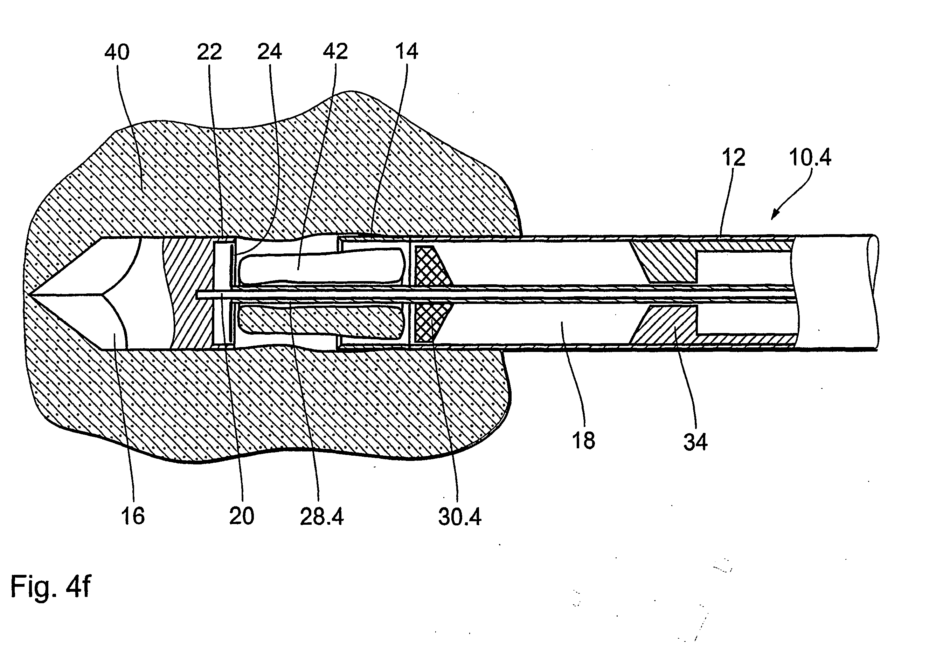

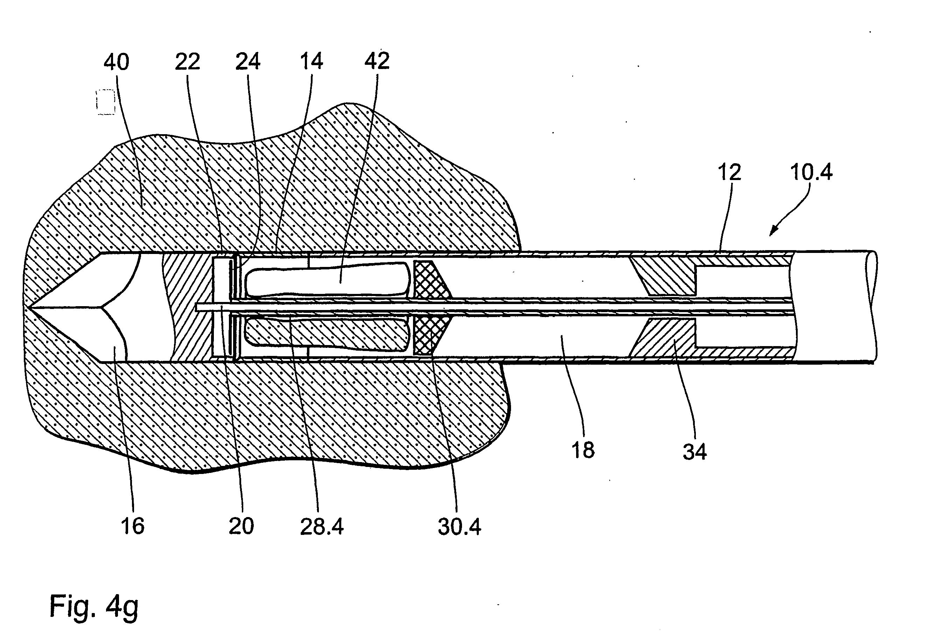

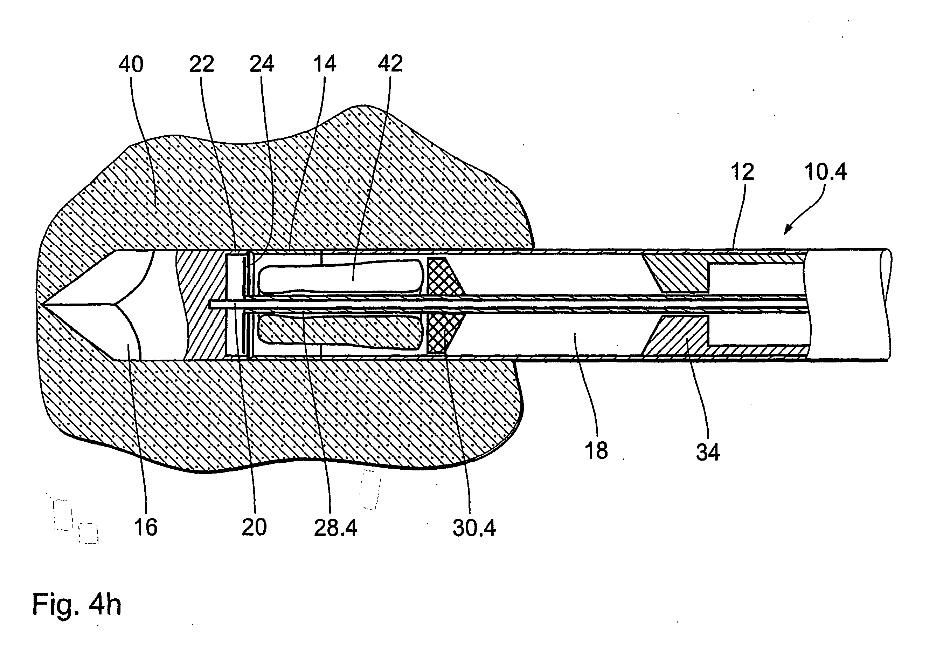

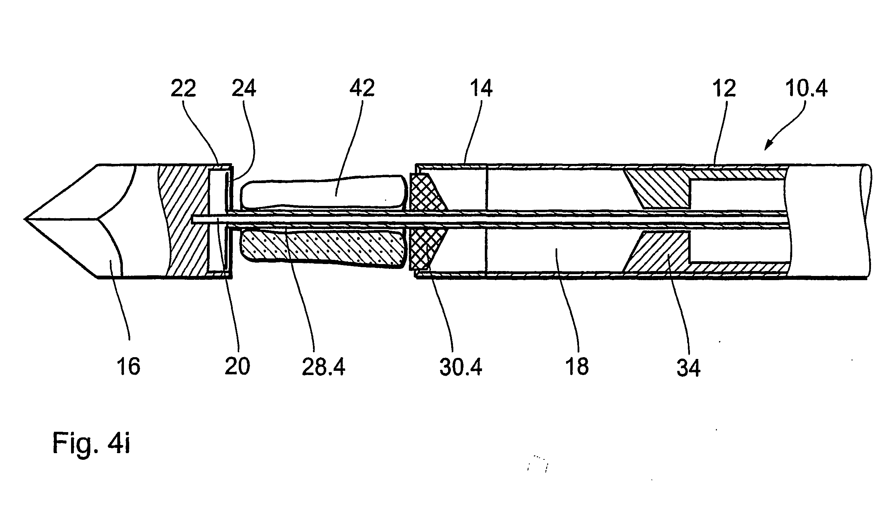

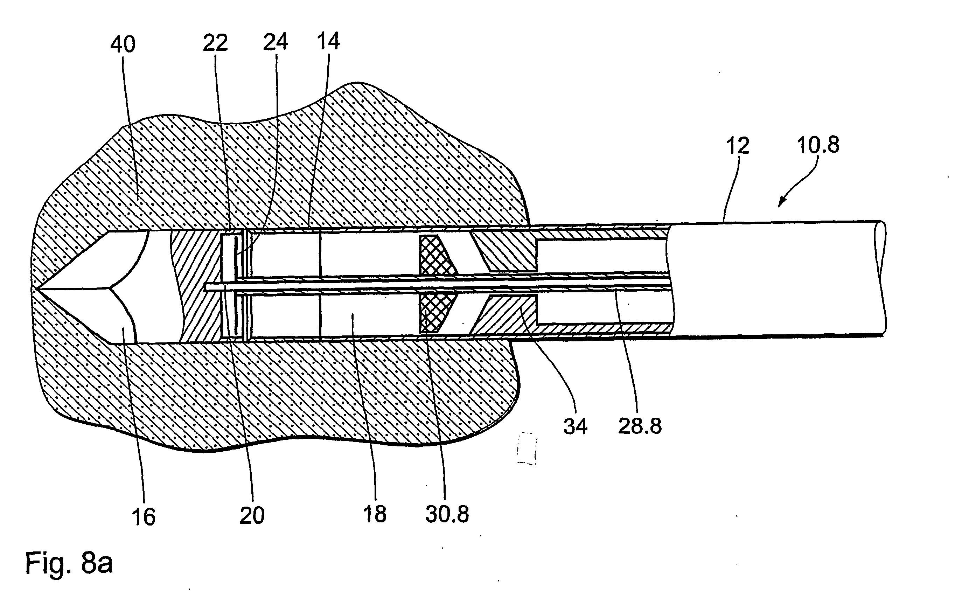

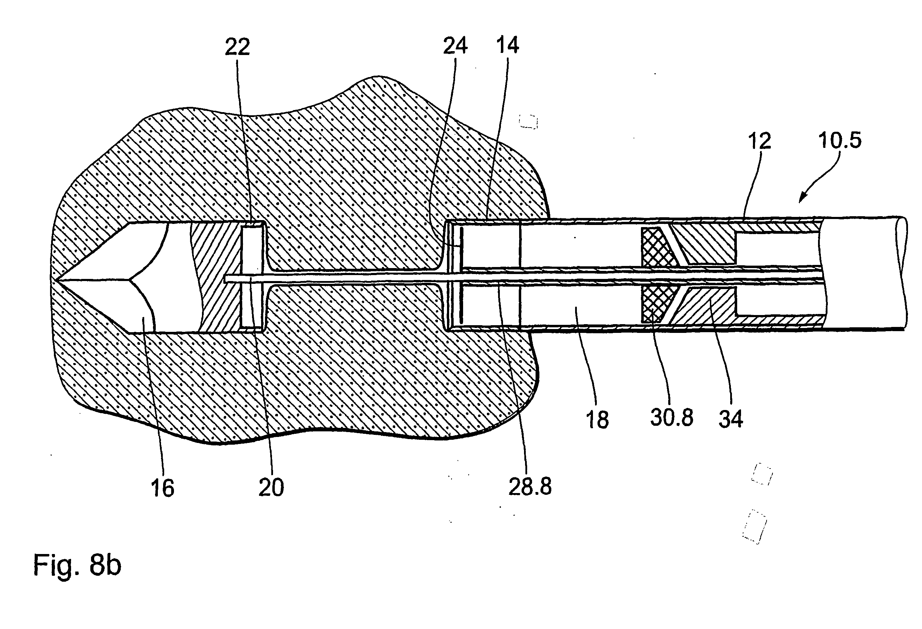

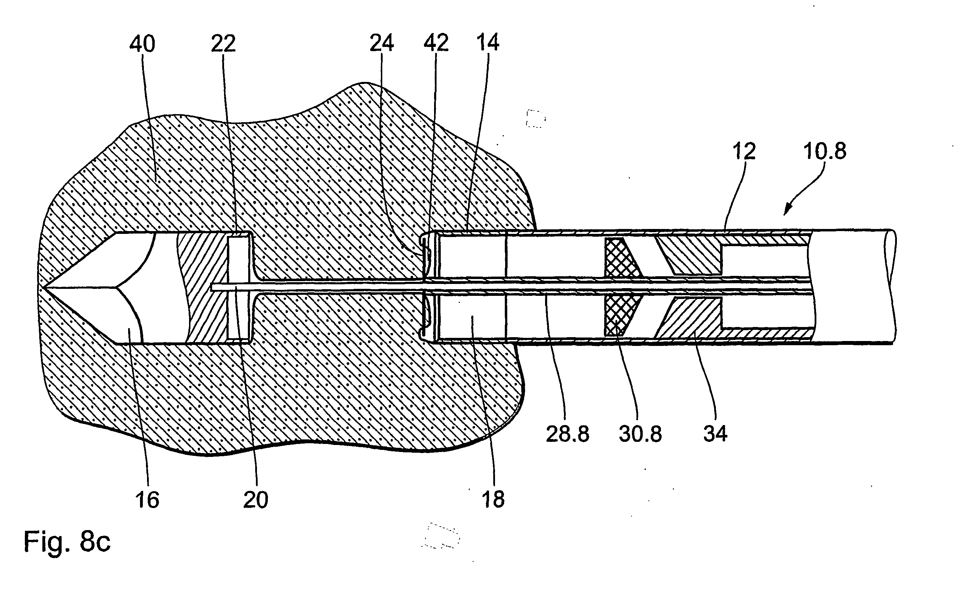

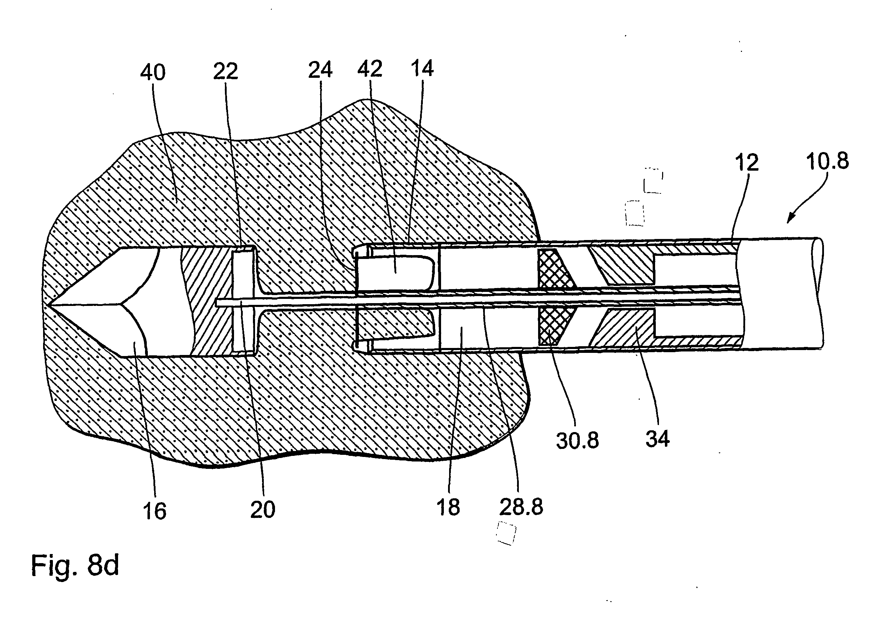

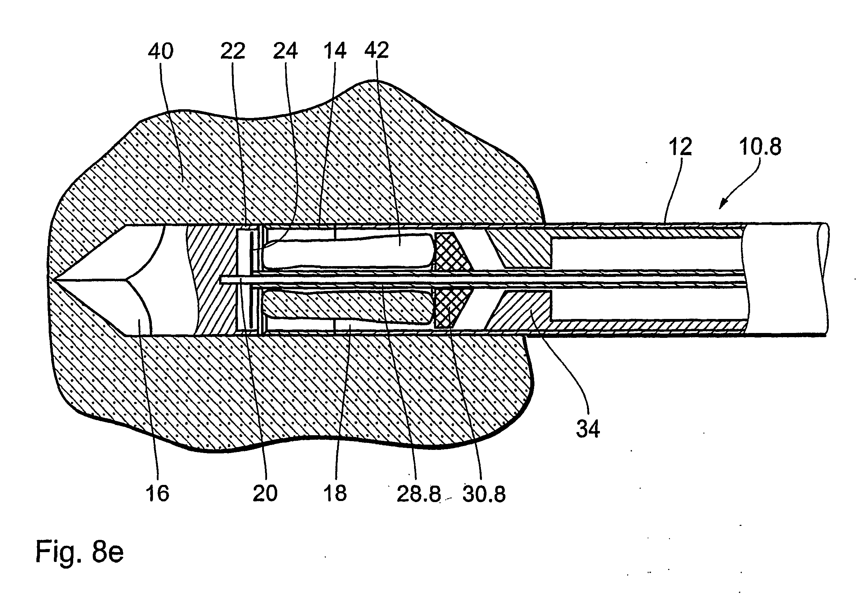

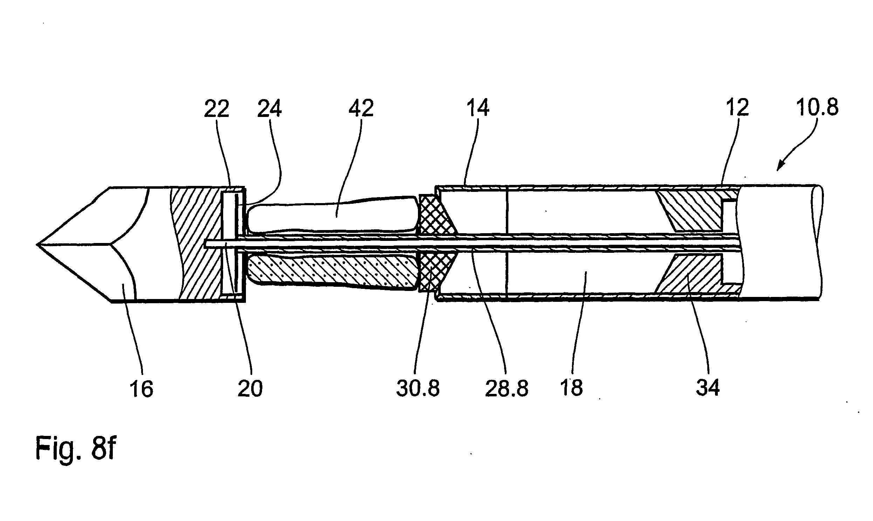

In den Figuren 8a bis 8f ist eine Hohlsonde 10.8 dargestellt, die in ihrem Aufbau der in den Figuren 4a bis 4j dargestellten Hohlsonde 10.4 ähnelt. Vorgesehen sind eine zentrale Schubstange 20 für die Metallspitze 16 und eine gemeinsame zentrale Schubstange 28 für das Schneidelement 24 und den Auswerfer 30. Der Aufnahmeraum 18 ist insgesamt kleiner als bei der in Figur 4 dargestellten Variante. Wie insbesondere den Figuren 8c und 8d zu entnehmen ist, werden nach dem Öffnen der Hohlsonde 10.8 die Metallhülse 12 sowie die Schubstange 28 mit dem Schneidelement 24 und dem Auswerfer 30 zusammen auf die Metallspitze 16 zu geschoben. Wichtig ist dabei, dass das Schneidelement 24 in - wenn auch geringem - Abstand zum distalen Ende der Metallhülse 12 angeordnet ist, so dass das Schneidelement 24 freisteht. Während die Metallhülse 12 und das Schneidelement 24 gemeinsam auf die Metallspitze 16 zu geschoben werden, schneidet das Schneidelement 24 einen Gewebeabschnitt 42 aus, der während des Vorschiebens der Metallhülse 12 gleich in den Aufnahmeraum 18 aufgenommen wird. Figur 8e zeigt die geschlossene Hohlsonde 10.8 nach dem vollständigen Abtrennen des Gewebeabschnittes 42. In Figur 8f ist die dem Gewebe 40 entnommene Hohlsonde 10.8 im geöffneten Zustand zum Auswerfen des abgetrennten Gewebeabschnittes 42 dargestellt.

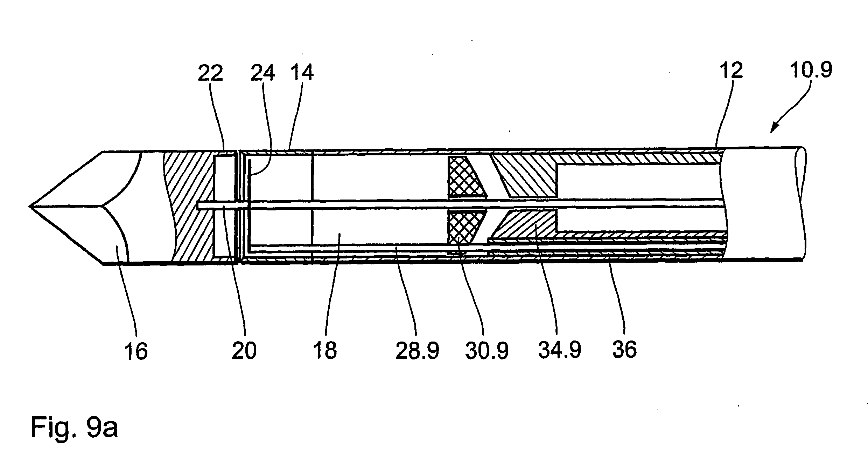

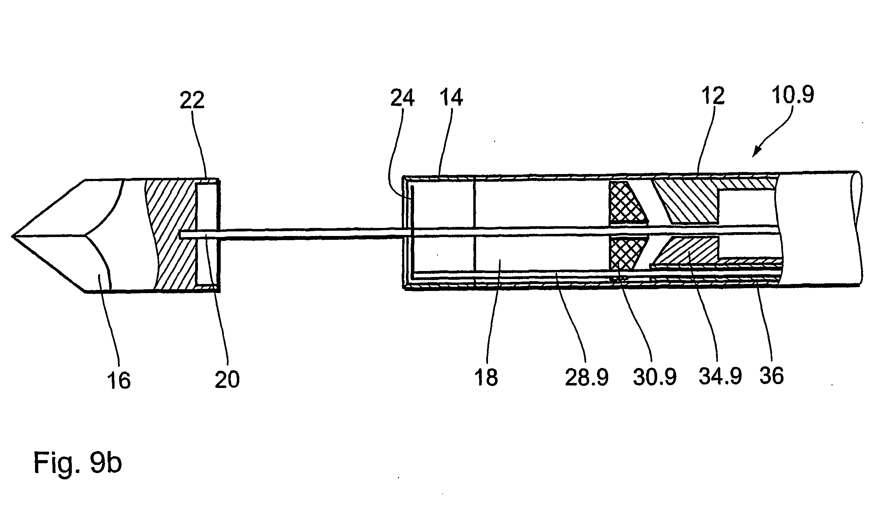

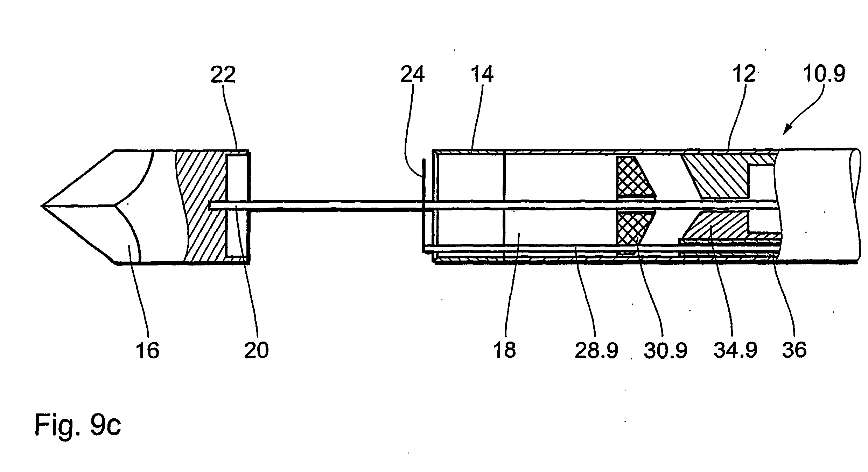

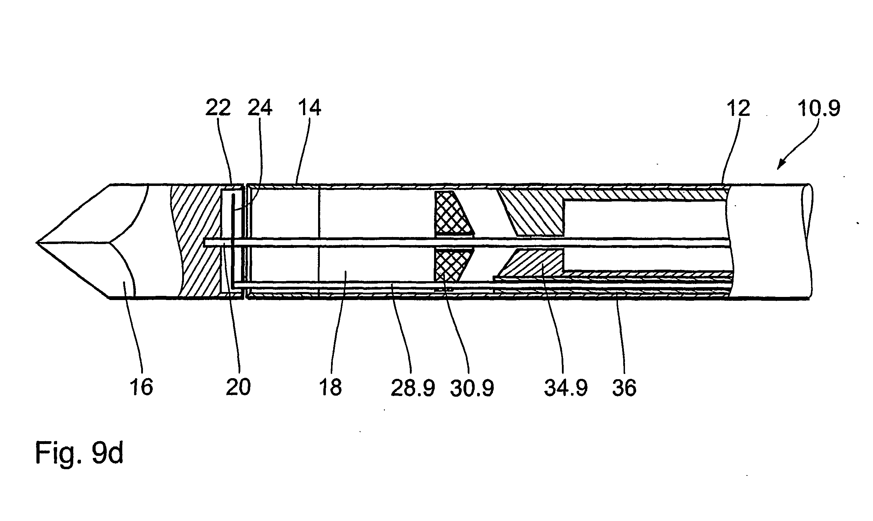

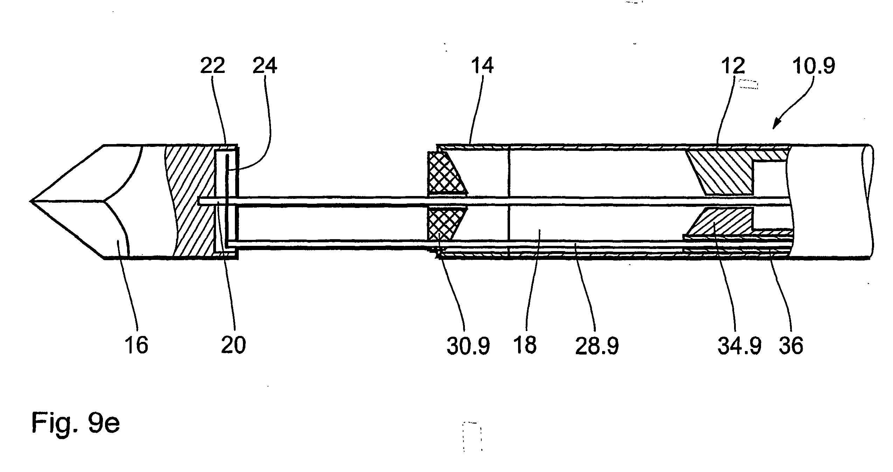

Bei der Variante einer Hohlsonde 10.9, abgebildet in den Figuren 9a bis 9e, ist statt einer gemeinsamen zentralen Schubstange für das Schneidelement 24 und den Auswerfer 30 eine gemeinsame dezentrale Schubstange 28.9 für das Schneidelement 24 und den Auswerfer 30 vorgesehen. Die Schubstange 28.9 ist in einer Führungshülse 36geführt. DerBewegungsablauf beim Abtrennen eines Gewebeabschnittes ist ähnlich wie bei der in Figur 8 dargestellten Variante. Im Unterschied zu dort ist das Schneidelement 24 beim Öffnen der Hohlsonde 10.9 zunächst in den Aufnahmeraum 18 zurückgezogen und wird dann zum Abtrennen des Gewebeabschnittes etwas in Richtung der Metallspitze 16 vorgeschoben, so dass das Schneidelement 24 freisteht; siehe Figur 9c. Anschließend werden das Schneidelement 24 zusammen mit dem Auswerfer 30 und der Metallhülse 12 auf die Metallspitze 16 zu geschoben, so dass ein Gewebeabschnitt auf die gleiche Weise abgetrennt wird, wie in den Figuren 8a bis 8e dargestellt. Figur 9d zeigt die geschlossene Sonde 10.9 nach dem Abtrennen eines Gewebeabschnittes, Figur 9e die zum Auswerfen eines Gewebeabschnittes geöffnete Hohlsonde 10.9 nach ihrer Entnahme aus dem Gewebe.

Die Figuren 10a bis 10h zeigen verschiedene Ausführungsvarianten eines mit einer zentralen Schubstange 28 verbundenen Schneidelementes 24. Zu erkennen sind jeweils eine oder mehrere Streben 26, mit denen das jeweilige Schneidelement 24 mit der jeweiligen Schubstange 28 verbunden ist. Die Streben 26 haben vorzugsweise den gleichen Durchmesser wie das von einem Drahtring gebildete Schneidelement 24. Der Drahtdurchmesser liegt zwischen 0,05 und 1,0 mm, vorzugsweise bei 0,15 mm. Die Schubstange 28 ist jeweils als Rohr ausgebildet, so dass in der Schubstange 28 eine weitere Schubstange für die Metallspitze der jeweiligen Hohlsonde geführt werden kann. Die Drahtstreben 26 haben nicht nur den Zweck, das Schneidelement 24 zu halten, sondern auch die Eigenschaft abgetrennte Gewebeabschnitte längs zu schneiden, wie zuvor beschrieben.

In den Figuren 11a bis 11h sind jeweils eine Schubstange 28 mit daran befestigten, von einem Drahtring gebildeten Schneidelement 24 dargestellt. Bei den dargestellen Varianten sind Drahtstreben zum Halten des Schneidelementes 24 an der Schubstange 28 offensichtlich nicht erforderlich. Die in den Figuren 11a bis 11h abgebildeten Drahtstreben 26 haben somit einzig den Zweck als Schneidelektrode zum Längsschlitzen eines abgetrennten Gewebeabschnittes zu dienen. Wie in den Figuren 11i bis 11k abgebildet, kann eine Drahtstrebe 26 auch als Drahtschlaufe ausgebildet sein. Die Vorderansicht der in den Figuren 11i bis 11k abgebildeten Drahtschlaufe 26 ist die gleiche wie die der Drahtstrebe 26 aus Figur 11a, so dass sich bei Bewegung der Schubstange 28 in Längsrichtung in beiden Fällen der gleiche Schnitt ergibt.

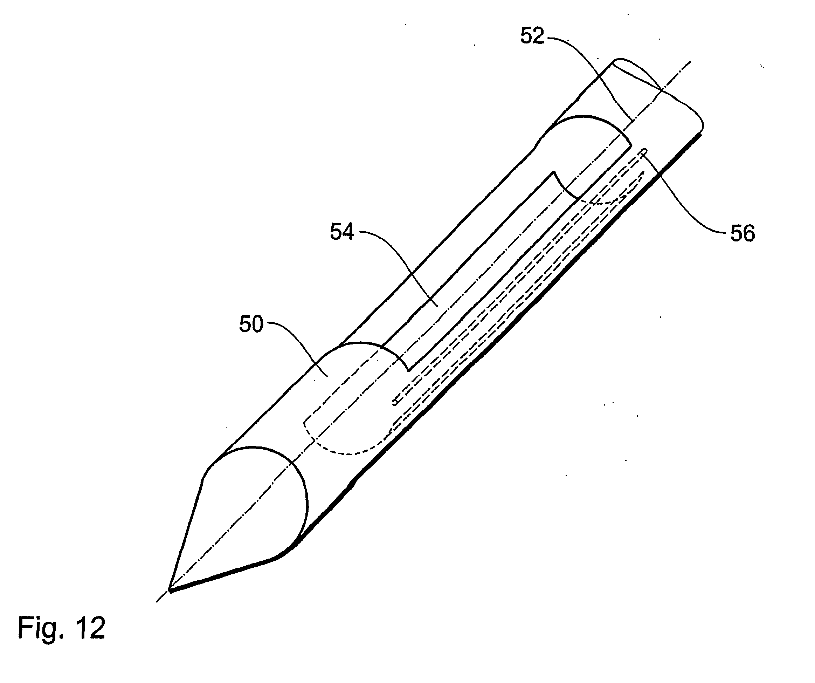

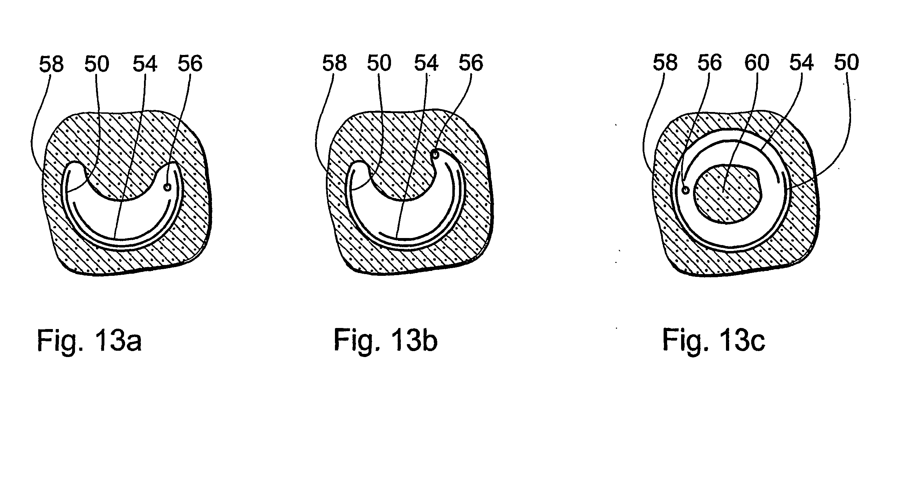

In Figur 12 ist eine alternative Hohlsonde 50 abgebildet, bei der ein zylinderförmiger Aufnahmeraum halbseitig nach außen geöffnet ist. Diese Öffnung ist durch einen um die Längsachse 52 der Hohlsonde 50 drehbaren Deckel 54 zu verschlie-ßen. Vor einer Deckellängskante ist ein parallel zur Längsachse 52 der Hohlsonde 50 ausgerichtetes gerades Schneidelement 56 angebracht. An die Hohlsonde 50 kann vorzugsweise Vakuum angelegt werden, mit dessen Hilfe Gewebe 58 in die geöffnete Hohlsonde 50 eingesaugt wird. Zum Verschließen der Öffnung wird der Deckel 54 mitsamt dem Schneidelement 56 um die Längsachse 52 der Hohlsonde 50 derart gedreht, dass das Schneidelement 56 dem Deckel 54 vorangeht und den in die Öffnung der Hohlsonde 50 eingesaugten Gewebeabschnitt 60 abtrennt. Dies ist in den Figuren 13a bis 13c dargestellt.

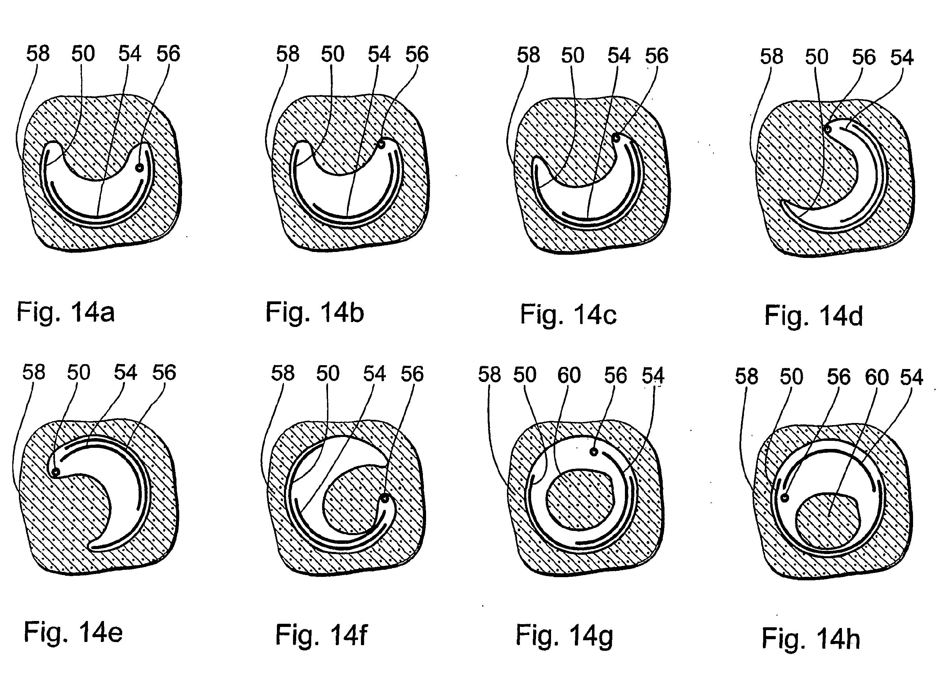

Um auf eine einfache Art und Weise einen größeren Gewebeabschnitt mit der in Figur 12 dargestellten Hohlsonde 50 abtrennen zu können, kann die geöffnete Hohlsonde 50 mit freistehendem Schneidelement 56 in dem Gewebe 58 um ihre Längsachse 52 gedreht werden, siehe Figuren 14a bis 14h, um so einen entsprechenden Gewebeabschnitt 60 auszuschälen. Anschließend wird die Hohlsonde 50 wieder geschlossen und dem Gewebe entnommen.

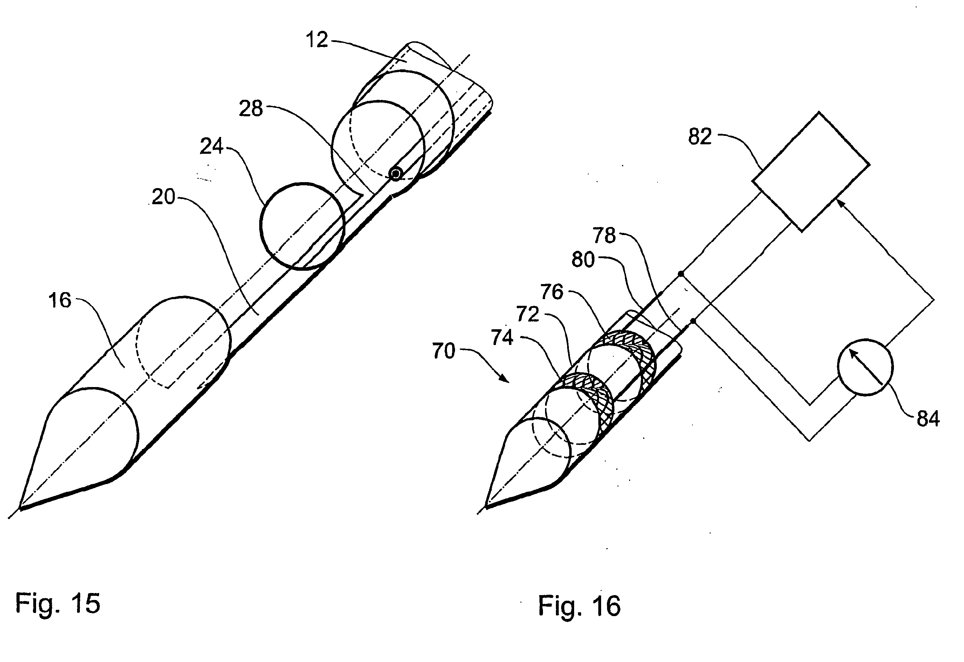

Figur 15 zeigt eine Variante der in den Figuren 3a bis 3e dargestellten Hohlsonde 10.3 in perspektivischer Darstellung.

In Figur 16 ist eine chirurgische Vorrichtung 70 mit einer Hohlsonde 72 dargestellt, welche mit zwei Elektroden 74 76 zum Einleiten hochfrequenten elektrischen Stroms in die Hohlsonde 72 umgebendes Gewebe ausgestattet ist. Die beiden Elektroden 74 und 76 sind über elektrische Leitungen 78 und 80 mit einem Generator 82 zum Erzeugen der ins Gewebe einzubringenden elektrischen Leistung verbunden. An die Leitungen angeschlossen ist außerdem eine Messeinheit 84, welche zum Bestimmen der Impedanz zwischen den beiden Elektroden 72 und 74 ausgebildet ist. Die Messeinrichtung 84 ist mit dem Generator 82 verbunden, um dessen Leistung in Abhängigkeit der zwischen den Elektroden 74 und 76 gemessenen Impedanz zu steuern. Durch diese Rückführung der Impedanzmesswerte ergibt sich insgesamt eine Generatorregelung. Die Steuerung des Generators 82 mittels des Impedanzsignals kann auch zum therapiegerechten Abschalten des Generators 82 herangezogen werden. Mit zunehmender Verödung des Gewebes steigt dessen Impedanz an. Daher kann ein Grenzwert für die Impedanz vorgesehen sein, von dem ab der Generator abgeschaltet wird, da die gemessene hohe spezifische Gewebeimpedanz anzeigt, dass das Gewebe ausreichend verödet ist.

## Patentansprüche

1. Chirurgische Hohlsonde (10) zur minimal invasiven Entnahme von Gewebe mit einem länglichen Hohlkörper, welcher zwei Segmente (12, 16) umfasst, von denen zumindest eines einen Hohlraum (18) zur Aufnahme von Gewebe einschließt und welche voneinander trennbar und relativ zueinander derart bewegbar angeordnet sind, dass eine Öffnung zwischen den Segmenten (12, 16) wahlweise freizugeben oder zu verschließen ist, **gekennzeichnet durch** ein elektrisch leitendes, relativ zu zumindest einem der Segmente bewegbares ring- oder schlaufenförmigem Schneidelement (24), das zum elektrochirurgischen Ausschneiden von in die Öffnung zwischen den Hohlkörpersegmenten (12, 16) eingedrungenem Gewebe und derart ausgebildet ist, dass das Schneidelement (24) vollständig, ins Innere des Hohlraums bewegt werden kann, wobei das Schneidelement (24) an einer Schubstange (28) befestigt ist, die so gelagert ist, dass das ring- oder schlaufenförmige Schneidelement (24) translatorisch ausschließlich in Längsrichtung des Hohlkörpers bewegt werden kann.

2. Hohlsonde nach Anspruch 1, **gekennzeichnet durch** einen Auswerfer (30) zum Auswerfen von Gewebe aus der Hohlsonde (10).

3. Hohlsonde nach Anspruch 1 oder 2, **dadurch gekennzeichnet, dass** das Schneidelement (24) freistehend oder in eine freistehende Position bewegbar ist.

4. Hohlsonde nach einem der Ansprüche 1 bis 3, **dadurch gekennzeichnet, dass** das Schneidelement (24) als Schneidelektrode zum Anlegen einer HF-Spannung elektrisch leitend ausgebildet ist.

5. Hohlsonde nach einem der Ansprüche 1 bis 4, **dadurch gekennzeichnet, dass** die beiden Segmente (12, 16) entlang einer Umfangslinie um den Hohlraum (18) voneinander trennbar sind.

6. Hohlsonde nach einem der Ansprüche 1 bis 5, **dadurch gekennzeichnet, dass** die beiden Segmente (12, 16) axial zueinander verschiebbar sind.

7. Hohlsonde nach Anspruch 6, **dadurch gekennzeichnet, dass** die Öffnung zwischen den Segmenten (12, 16) je nach Querschnittsform des Hohlraums (18) zylinder- oder prismenförmig ist.

8. Hohlsonde nach einem der Ansprüche 1 bis 7, **dadurch gekennzeichnet, dass** die beiden Segmente (12, 16) über eine in einem der Segmente geführte Schubstange (20) miteinander verbunden sind.

9. Hohlsonde nach Anspruch 8, **dadurch gekennzeichnet, dass** die Schubstange (20) mittig in dem von den Segmenten (12, 16) eingeschlossenen Hohlraum (18) verläuft.

10. Hohlsonde nach einem der Ansprüche 4 bis 9, **dadurch gekennzeichnet, dass** das Schneidelement axial verschiebbar ist und eine elektrisch leitende Schlaufe (24) aufweist, die zum Ausschneiden eines Zylinder- oder prismenförmigen Gewebeabschnitts geformt ist.

11. Hohlsonde nach Anspruch 9 und 10, **dadurch gekennzeichnet, dass** das Schneidelement einen Schneidsteg (26) aufweist, der sich zwischen der Schubstange (20) und der Schneidschlaufe (24) erstreckt und mit der Schneidschlaufe (24) elektrisch leitend verbunden ist.

12. Hohlsonde nach einem der Ansprüche 1 bis 11, **dadurch gekennzeichnet, dass** das Schneidelement (24) innerhalb des Hohlraums verschiebbar ist.

13. Hohlsonde nach einem der Ansprüche 1 bis 12, **dadurch gekennzeichnet, dass** das Schneidelement (24) innerhalb der Öffnung verschiebbar ist.

14. Hohlsonde nach einem der Ansprüche 1 bis 13, **dadurch gekennzeichnet, dass** die beiden relativ zueinander bewegbaren Segmente (12, 16) als Elektroden zum Anlegen einer HF-Spannung für das thermische Inaktivieren von Gewebe ausgebildet sind.

15. Hohlsonde nach einem der Ansprüche 1 bis 14, **dadurch gekennzeichnet, dass** die beiden Segmente (12, 16) nahe des distalen Endes der Hohlsonde (10) voneinander getrennt sind.

16. Hohlsonde nach einem der Ansprüche 1 bis 15, **dadurch gekennzeichnet, dass** eines der Segmente (16) eine angeschärfte Metallspitze am distalen Ende der Hohlsonde (10) bildet.

17. Hohlsonde nach einem der Ansprüche 2 bis 14, **dadurch gekennzeichnet, dass** die Hohlsonde einen Auswerfer (30) aufweist, der innerhalb des Hohlraums (18) axial verschiebbar angeordnet ist.

18. Hohlsonde nach einem der Ansprüche 2 bis 15, **dadurch gekennzeichnet, dass** die Hohlsonde einen Auswerfer aufweist, der aus einer an einem Ende des Hohlraums (18) befindlichen Ruheposition in Richtung des Hohlraums (18) verschiebbar angeordnet ist und auf seiner in Ruheposition dem Hohlraum (18) zugewandten Seite ein dem Hohlraum (18) zugewandtes Trennmesser trägt.

19. Hohlsonde nach einem der Ansprüche 2 bis 16, **dadurch gekennzeichnet, dass** die Hohlsonde einen Auswerfer aufweist, der mit dem Schneidelement (24) an einer gemeinsamen Schubstange (28) befestigt ist, welche bezüglich der Segmente (12, 16) längsverschieblich geführt ist.

20. Chirurgische Vorrichtung mit einer Hohlsonde (72) nach Anspruch 14, **dadurch gekennzeichnet, dass** zwei Elektroden (74, 76) der Hohlsonde (72) zum Abgeben elektrischer Energie an die Hohlsonde (72) umgebendes Gewebe ausgebildet und mit einer Messvorrichtung (84) verbunden sind, die zum Erzeugen eines von der Impedanz zwischen den beiden Elektroden (74, 76) abhängigen Signals ausgebildet ist.

21. Chirurgische Vorrichtung nach Anspruch 20, **dadurch gekennzeichnet, dass** die Messvorrichtung (84) mit einem Generator (82) für über die Elektroden (74, 76) abzugebende elektrische Energie derart verbunden ist, dass eine von dem Generator (82) abgegebene elektrische Leistung durch das von der Messvorrichtung (84) erzeugte Signal steuerbar ist.

## Claims

1. Hollow surgical probe (10) for the minimally invasive removal of tissue comprising an elongated hollow body which includes two segments (12, 16) of which at least one encloses a cavity (18) for receiving tissue and which can be separated from each other and are arranged movably relative to each other in such a way that an opening between the segments (12, 16) can be selectively opened or closed, **characterised by** an electrically conductive ring-shaped or loop-shaped cutting element (24) which is movable relative to at least one of the segments and is adapted for electrosurgically cutting out tissue which has penetrated into the opening between the hollow body segments (12, 16) in such a way that the cutting element can be moved completely inside the cavity, the cutting element (24) being connected to a thrust rod (28), which is mounted in such a way that the ring-shaped or loop-shaped cutting element (24) can be moved translatorily and exclusively in the longitudinal direction of the hollow body.

2. Hollow probe according to claim 1, **characterised by** an ejector (30) for ejecting tissue from the hollow probe (10).

3. Hollow probe according to either claim 1 or claim 2, **characterised in that** the cutting element (24) is exposed or is movable into an exposed position.

4. Hollow probe according to any one of claims 1 to 3, **characterised in that** the cutting element (24) is electrically conductive in the form of a cutting electrode for the application of an HF voltage.

5. Hollow probe according to any one of claims 1 to 4, **characterised in that** the two segments (12, 16) are separable from each other along a peripheral line around the cavity (18).

6. Hollow probe according to any one of claims 1 to 5, **characterised in that** the two segments (12, 16) are axially displaceable relative to each other.

7. Hollow probe according to claim 6, **characterised in that** the opening between the segments (12, 16) is cylindrical or prism-shaped depending on the respective cross-sectional shape of the cavity (18).

8. Hollow probe according to any one of claims 1 to 7, **characterised in that** the two segments (12, 16) are joined together by way of a thrust rod (20) guided in one of the segments.

9. Hollow probe according to claim 8, **characterised in that** the thrust rod (20) extends centrally in the cavity (18) enclosed by the segments (12, 16).

10. Hollow probe according to any one of claims 4 to 9, **characterised in that** the cutting element is axially displaceable and has an electrically conductive loop (24) which is shaped to cut out a cylindrical or prism-shaped portion of tissue.

11. Hollow probe according to claims 9 and 10, **characterised in that** the cutting element has a cutting arm (26) which extends between the thrust rod (20) and the cutting loop (24) and is electrically conductively connected to the cutting loop (24).

12. Hollow probe according to any one of claims 1 to 11, **characterised in that** the cutting element (24) is displaceable within the cavity.

13. Hollow probe according to any one of claims 1 to 12, **characterised in that** the cutting element (24) is displaceable within the opening.

14. Hollow probe according to any one of claims 1 to 13, **characterised in that** the two segments (12, 16) which are movable relative to each other are in the form of electrodes for the application of an HF voltage for thermal inactivation of tissue.

15. Hollow probe according to any one of claims 1 to 14, **characterised in that** the two segments (12, 16) are separated from each other near the distal end of the hollow probe (10).

16. Hollow probe according to any one of claims 1 to 15, **characterised in that** one of the segments (16) forms a sharpened metal tip at the distal end of the hollow probe (10).

17. Hollow probe according to any one of claims 2 to 14, **characterised in that** the hollow probe has an ejector (30) which is arranged axially displaceably within the cavity (18).

18. Hollow probe according to any one of claims 2 to 15, **characterised in that** the hollow probe has an ejector which is arranged displaceably out of a rest position at one end of the cavity (18) in the direction of the cavity (18) and on its side facing the cavity (18) in the rest position carries a severing blade facing the cavity (18).

19. Hollow probe according to any one of claims 2 to 16, **characterised in that** the hollow probe has an ejector, which, with the cutting element (24), is fixed to a common thrust rod (28) which is guided so as to be longitudinally displaceable with respect to the segments (12, 16).

20. Surgical apparatus comprising a hollow probe (72) according to claim 14, **characterised in that** two electrodes (74, 76) of the hollow probe (72) are adapted to deliver electrical energy to tissue surrounding the hollow probe (72), and are connected to a measuring device (84) which is adapted to produce a signal dependent on the impedance between the two electrodes (74, 76).

21. Surgical apparatus according to claim 20, **characterised in that** the measuring device (84) is connected to a generator (82) for electrical energy to be delivered by way of the electrodes (74, 76), in such a way that electrical power delivered by the generator (82) is controllable by the signal produced by the measuring device (84).

## Revendications

1. Sonde chirurgicale creuse (10) pour le prélèvement mini-invasif de tissu, comportant un corps creux longiligne, qui est formé de deux segments (12, 16), dont au moins un contient une cavité (18) destinée à recevoir le tissu et lesquels peuvent être séparés l'un de l'autre et être déplacés l'un vers l'autre de manière à libérer ou à fermer au choix une ouverture entre les segments (12, 16), **caractérisée par** un élément de coupe (24) électroconducteur en forme d'anneau ou de boucle, qui peut être déplacé par rapport à au moins un des segments et qui est conçu pour l'ablation électro-chirurgicale du tissu engagé dans l'ouverture entre les segments (12, 16) du corps creux et de telle sorte que l'élément de coupe (24) peut être déplacé entièrement à l'intérieur de la cavité, l'élément de coupe (24) étant fixé contre une tige de poussée (28), qui est montée de telle sorte que l'élément de coupe (24) en forme d'anneau ou de boucle peut être déplacé en translation exclusivement dans le sens longitudinal du corps creux.

2. Sonde creuse selon la revendication 1, **caractérisée par** un éjecteur (30) destiné à éjecter le tissu hors de la sonde creuse (10).

3. Sonde creuse selon la revendication 1 ou 2, **caractérisée en ce que** l'élément de coupe (24) est isolé ou peut être amené dans une position isolée.

4. Sonde creuse selon l'une quelconque des revendications 1 à 3, **caractérisée en ce que** l'élément de coupe (24) est réalisé sous la forme d'une électrode de coupe électroconductrice, destinée à appliquer une tension de haute fréquence.

5. Sonde creuse selon l'une quelconque des revendications 1 à 4, **caractérisée en ce que** les deux segments (12, 16) peuvent être séparés l'un de l'autre le long d'une ligne périphérique autour de la cavité (18).

6. Sonde creuse selon l'une quelconque des revendications 1 à 5, **caractérisée en ce que** les deux segments (12, 16) sont mobiles l'un par rapport à l'autre dans le sens axial.

7. Sonde creuse selon la revendication 6, **caractérisée en ce que** l'ouverture entre les segments (12, 16) est cylindrique ou prismatique en fonction de la forme de la section de la cavité (18).

8. Sonde creuse selon l'une quelconque des revendications 1 à 7, **caractérisée en ce que** les deux segments (12, 16) sont reliés l'un à l'autre par l'intermédiaire d'une tige de poussée (20) logée dans l'un des segments.

9. Sonde creuse selon la revendication 8, **caractérisée en ce que** la tige de poussée (20) s'étend au centre dans la cavité (18) contenue dans les segments (12, 16).

10. Sonde creuse selon l'une quelconque des revendications 4 à 9, **caractérisée en ce que** l'élément de coupe est mobile dans le sens axial et comporte une boucle (24) électroconductrice, qui est formée pour l'ablation d'un morceau de tissu cylindrique ou prismatique.

11. Sonde creuse selon les revendications 9 et 10, **caractérisée en ce que** l'élément de coupe comporte une barrette de coupe (26) qui s'étend entre la tige de poussée (20) et la boucle de coupe (24) et qui est reliée de manière électroconductrice à la boucle de coupe (24).

12. Sonde creuse selon l'une quelconque des revendications 1 à 11, **caractérisée en ce que** l'élément de coupe (24) est mobile à l'intérieur de la cavité.

13. Sonde creuse selon l'une quelconque des revendications 1 à 12, **caractérisée en ce que** l'élément de coupe (24) est mobile à l'intérieur de l'ouverture.

14. Sonde creuse selon l'une quelconque des revendications 1 à 13, **caractérisée en ce que** les deux segments (12, 16), mobiles l'un par rapport à l'autre, sont réalisés sous forme d'électrodes, destinées à appliquer une tension de haute fréquence pour l'inactivation thermique du tissu.

15. Sonde creuse selon l'une quelconque des revendications 1 à 14, **caractérisée en ce que** les deux segments (12, 16) sont séparés l'un de l'autre à proximité de l'extrémité distale de la sonde creuse (10).

16. Sonde creuse selon l'une quelconque des revendications 1 à 15, **caractérisée en ce que** l'un des segments (16) forme une pointe métallique chanfreinée sur l'extrémité distale de la sonde creuse (10).

17. Sonde creuse selon l'une quelconque des revendications 2 à 14, **caractérisée en ce que** la sonde creuse comporte un éjecteur (30) qui est monté mobile dans le sens axial à l'intérieur de la cavité (18).

18. Sonde creuse selon l'une quelconque des revendications 2 à 15, **caractérisée en ce que** la sonde creuse comporte un éjecteur, qui est monté de manière à pouvoir être déplacé hors d'une position de repos, située à une extrémité de la cavité (18), vers la cavité (18) et porte, sur son côté orienté vers la cavité (18) dans la position de repos, une lame de coupe orientée vers la cavité (18).

19. Sonde creuse selon l'une quelconque des revendications 2 à 16, **caractérisée en ce que** la sonde creuse comporte un éjecteur qui est fixé avec l'élément de coupe (24) contre une tige de poussée (28) commune, qui est montée mobile dans le sens longitudinal par rapport aux segments (12, 16).

20. Dispositif chirurgical comportant une sonde creuse (72) selon la revendication 14, **caractérisé en ce que** deux électrodes (74, 76) de la sonde creuse (72) sont réalisées pour fournir de l'énergie électrique au tissu entourant la sonde creuse (72) et sont reliées à un dispositif de mesure (84) qui est réalisé pour générer un signal dépendant de l'impédance entre les deux électrodes (74, 76).

21. Dispositif chirurgical selon la revendication 20, **caractérisé en ce que** le dispositif de mesure (84) est relié à un générateur (82) pour l'énergie électrique à fournir aux électrodes (74, 76), de telle sorte qu'une puissance électrique fournie par le générateur (82) peut être commandée par le signal généré par le dispositif de mesure (84).
